# EUROPEAN PATENT SPECIFICATION

(11) **EP 3 966 214 B1**
(45) Date of publication and mention of the grant of the patent: **13.12.2023**
(21) Application number: 20724678.6
(22) Date of filing: 17.04.2020
(51) Int. Cl.: C07D 487/04, A61F 13/42

(54) **SYNTHESIS OF COELENTERAZINE**
SYNTHESE VON COELENTERAZIN
SYNTHÈSE DE COELENTÉRAZINE

(30) Priority: 08.05.2019 US 201962845189 P; 28.06.2019 US 201916457788; 28.06.2019 US 201916457732
(43) Date of publication of application: 16.03.2022
(73) Proprietor: International Paper Company, Memphis, TN 38197 (US)
(72) Inventor: DUVVURU, Rajagopala Reddy, Hyderabad, TS 500034 (IN)
(74) Representative: Fish & Richardson P.C.
(86) International application number: PCT/US2020/028785
(87) International publication number: WO 2020/226883

(56) References cited:
- MACIEJ ADAMCZYK ET AL: "SYNTHESIS OF COELENTERAZINE", ORGANIC PREPARATIONS AND PROCEDURES INTERNATIONAL: THE NEW JOURNAL FOR ORGANIC SYNTHESIS, vol. 33, no. 5, 1 October 2001 (2001-10-01), pages 477-485, XP055619683, US ISSN: 0030-4948, DOI: 10.1080/00304940109356615
- LU YUETING ET AL: "Efficient synthesis and antioxidant activity of coelenterazine analogues", TETRAHEDRON LETTERS, vol. 55, no. 45, 2014, pages 6212-6215, XP029080070, ISSN: 0040-4039, DOI: 10.1016/J.TETLET.2014.09.065
- SHIMOMURA O ET AL: "SEMI-SYNTHETIC AEQUORINS WITH IMPROVED SENSITIVITY TO CA2+ IONS", BIOCHEMICAL JOURNAL, PUBLISHED BY PORTLAND PRESS ON BEHALF OF THE BIOCHEMICAL SOCIETY, vol. 261, no. 3, 1 August 1989 (1989-08-01), pages 913-920, XP001007915, ISSN: 0264-6021
- VITO VECE ET AL: "Multicomponent synthesis of novel coelenterazine derivatives substituted at the C-3 position", TETRAHEDRON, vol. 71, no. 46, 1 November 2015 (2015-11-01), pages 8781-8785, XP055619709, AMSTERDAM, NL ISSN: 0040-4020, DOI: 10.1016/j.tet.2015.09.048
- MOSRIN MARC ET AL: "Regio- and Chemoselective Multiple Functionalization of Chloropyrazine Derivatives. Application to the Synthesis of Coelenterazine", ORGANIC LETTERS, AMERICAN CHEMICAL SOCIETY, US, vol. 11, no. 15, 8 June 2009 (2009-06-08), pages 3406-3409, XP009131219, ISSN: 1523-7060

## Description

This application claims the benefit of U.S. Application No. 16/851,962, filed on April 17, 2020, which claims the benefit of U.S. Application No. 16/457,788, filed on June 28, 2019, which claims the benefit of U.S. Provisional Application No. 62/845,189, filed May 8, 2019, and U.S. Provisional Application No. 62/692,485, filed June 29, 2018; and which also claims the benefit of U.S. Application No. 16/457,732, filed June 28, 2019, which claims the benefit the benefit of U.S. Provisional Application No. 62/753,024, filed October 30, 2018, and U.S. Provisional Application No. 62/692,502, filed June 29, 2018.

### BACKGROUND

Personal care absorbent products, such as infant diapers, adult incontinent pads, and feminine care products, typically contain a fluid absorbent core. Many absorbent articles include the fluid absorbent core disposed between a top sheet and a back sheet. The top sheet can be formed from a fluid-permeable material adapted to promote fluid transfer into the absorbent core, such as upon a liquid insult, usually with minimal fluid retention by the top sheet. U.S. southern pine fluff pulp is commonly used in the absorbent core, generally in the form of a fibrous matrix, and sometimes in conjunction with a superabsorbent polymer (SAP) dispersed throughout the fibrous matrix. This fluff pulp is recognized worldwide as the preferred fiber for absorbent products, based on factors such as the fluff pulp's high fiber length, fiber coarseness, and its relative ease of processing from a wet-laid and dried pulp sheet to an air-laid web. The raw material for this type of cellulosic fluff pulp is Southern Pine (e.g., Loblolly Pine, *Pinus taeda* L.,). The raw material is renewable, and the pulp is easily biodegradable. Compared to SAP, these fibers are inexpensive on a per mass basis but tend to be more expensive on per unit of liquid held basis. These fluff pulp fibers mostly absorb within the interstices between fibers. For this reason, a fibrous matrix readily releases acquired liquid on application of pressure. The tendency to release acquired liquid can result in significant skin wetness during use of an absorbent product that includes a core formed exclusively from cellulosic fibers. Such products also tend to leak the acquired liquid because liquid is not effectively retained in such a fibrous absorbent core.

SAPs are water-swellable, generally water-insoluble absorbent materials having a high absorbent capacity for fluids. They are used in absorbent articles like baby diapers or adult incontinent products to absorb and hold body fluids. SAP, upon absorption of fluids, swells and becomes a gel holding more than its weight of such fluids. The SAPs in common use are mostly derived from acrylic acid. Acrylic acid-based polymers also comprise a meaningful portion of the cost structure of diapers and incontinent pads. SAPs are designed to have high gel strength (as demonstrated by high absorbency under load or AUL). The high gel strength (upon swelling) of currently used SAP particles helps them to retain significant void space between particles, which is helpful for rapid fluid uptake. However, this high "void volume" simultaneously results in significant interstitial (between particles) liquid in the product in the saturated state. When there is interstitial liquid the "rewet" value or "wet feeling" of an absorbent product is compromised.

Some absorbent articles, such as diapers or adult incontinence pads, also include an acquisition and distribution layer (ADL) for the collection, and uniform and timely distribution of fluid from a fluid insult to the absorbent core. An ADL is usually placed between the top sheet and the absorbent core, and normally takes the form of composite fabric with most likely the top-one third of the fabric having low density (higher denier fiber) with relatively large voids and higher void volume for the effective acquisition of the presented fluid, even at relatively higher discharge rates. The middle one-third of the composite fabric of the ADL is usually made of higher density (low denier) fibers with smaller voids, while the lower one-third of the fabric is made of even higher density (lower and smaller denier) fibers and yet with finer voids. The higher density portions of the composite have more and finer capillaries and hence develop greater capillary pressure, thus moving greater volumes of fluid to the outer regions of the structure thus enabling the proper channelization and distribution of the fluid in an evenly fashion to allow the absorbent core to take up all of the liquid insult in a time bound manner to allow SAP within the absorbent core to hold and to gel the insult neither too slow nor too fast. The ADL provides for more rapid liquid acquisition (minimizing flooding in the target zone), and ensures more rapid transport and thorough distribution of the fluid into the absorbent core.

As noted above, the absorbent core is adapted to retain fluid, and as such may consist of one or more layers, such as layers to acquire, distribute, and/or store fluid. In many cases, a matrix of cellulose fibers, such as in the form of an air-laid pad and/or non-woven web, is used in (or as) the absorbent core of absorbent articles. In some cases, the different layers may consist of one or more different types of cellulose fibers, such as cross-linked cellulose fibers. The absorbent core may also include one or more fluid retention agents, such as one or more SAPs, distributed throughout the fiber matrix, usually as particles. Advances in SAP technology have allowed the design of absorbent core configurations in which fluff pulp contributes less to the absorbent capability of the core and more to providing a matrix structure in which the SAP is stably held. Fluff pulp fibers also provide fluid distribution functionality, to direct fluid to the SAP. However, it has been found that these structural and fluid distribution functions may be provided, in some configurations, by synthetic fibers, leading to the development of absorbent cores containing both fluff pulp fibers and synthetic fibers, and even "fluff-less" absorbent cores containing no fluff pulp fibers. These configurations may offer the advantage of being less physically bulky, without sacrificing absorbency.

The back sheet is typically formed from a fluid-impermeable material to form a barrier to prevent retained fluid from escaping.

Whatever the structure, when the absorbent article is wet from one or more liquid insults, the chances for the fluid coming in contact with the skin increases profoundly, and if left unchanged for a long time can result in diaper rash for infants or dermatitis problem in adults, thereby posing a skin wellness hazard. However, in general, the only way to know whether the diaper or the incontinent pad is dry or wet is to physically inspect it. During day time this may not pose a significant problem because a caregiver can check the diapers or adult incontinent products as many times as desired. However, inspections during night time can be a discomfort to the baby as well as to the adult, disturbing their sleep. Moreover, frequent night time inspections, such as several times in a single night, can disrupt the wearer's sleeping pattern, which poses health hazard to baby as well as the adult patient.

In addition, it is typical that an article of clothing, such as pants, pajamas, and/or undergarments, is worn over the diaper or absorbent article. Accordingly, even absorbent articles that incorporate different types of wetness and/or moisture indicators pose difficulties in timely discovery of an insult.

As a result, there it typically a time lapse between the insult and its discovery. If this time period is prolonged, then there exists the possibility of developing diaper rash, skin irritation, and/or skin flaking. These conditions can be very painful for those affected. This is particularly true for babies and those adults in care-giving facilities, and particularly true for night time insults, which can lead to longer periods prior to changing the absorbent article.

Previous moisture indicators incorporated into absorbent articles use color change as a visual indication of wetness detection. Inks that appear, or disappear, based on contact with liquid are popular mechanisms for wetness detection. Fluorescence has also been used for wetness detection, such as by incorporating a compound that fluoresces in the presence of a liquid. The mechanisms for such indicators generally fall into three broad categories:
(1) imprinting a moisture indicating pattern on one of the piles of the absorbent article;
(2) discrete moisture- indicating strips or layers that are incorporated between the layers of the absorbent article; and (3) a discrete (i.e., not part of the absorbent article's construction) indicating strip that is fastened to the interior of the absorbent article immediately prior to use.

Whatever the mechanism, these visual indicators are deficient in low-light (e.g., night time) situations. Appearing or disappearing inks must be directly visually detected, such that the caregiver can see the absorbent product. In low-light situations, this may require both a light source (e.g., overhead light or flashlight), as well as the removal of covering garments (e.g., pajamas or undergarments). Fluorescent indicators suffer similar issues, in that they require an external light source to excite the fluorescent compound. Such excitation is typically provided by exposing the indicator to UV light (which presents health concerns to the wearer and caregiver) and must be in direct optical communication with the fluorescent compound, which then requires removal of covering garments, blankets, etc. Therefore, the use of visual indicators previously used to detect wetness in absorbent garments suffers many disadvantages in low-light situations, which greatly reduces the usefulness of their indication mechanisms.

There is a need for chemiluminescent materials that can generate light upon exposure to moisture, where the chemiluminescent materials are readily synthesized in large yields and good purity. The present disclosure seeks to fulfill these needs and provides further advantages.

### SUMMARY

This summary is provided to introduce a selection of concepts in a simplified form that are further described below in the Detailed Description. This summary is not intended to identify key features of the claimed subject matter, nor is it intended to be used as an aid in determining the scope of the claimed subject matter.

In one aspect, the present disclosure features a method of making coelenterazine, including: coupling 4-(5-amino-6-benzylpyrazin-2-yl)phenol (coelenteramine) with 3-(4-(benzyloxy)phenyl)-2-oxopropanal to provide 8-benzyl-2-(4-(benzyloxy)benzyl)-6-(4-hydroxyphenyl)imidazo[1,2-α]pyrazin-3(7*H*)-one; and deprotecting the 8-benzyl-2-(4-(benzyloxy)benzyl)-6-(4-hydroxyphenyl)imidazo[1,2-α]pyrazin-3(7*H*)-one to provide 8-benzyl-2-(4-hydroxybenzyl)-6-(4-hydroxyphenyl)imidazo[1,2-α]pyrazin-3(7*H*)-one (coelenterazine).

In another aspect, the present disclosure features a method of making 3-(4-(benzyloxy)phenyl)-2-oxopropanal, including providing 1-(benzyloxy)-4-(chloromethyl)benzene, and reacting the 1-(benzyloxy)-4-(chloromethyl)benzene in two steps to provide 3-(4-(benzyloxy)phenyl)-2-oxopropanal.

In another aspect, the present disclosure features a method of making an absorbent article, including incorporating the coelenterazine made according to the methods of the present disclosure into an absorbent article.

In yet another aspect, the present disclosure features an absorbent article, including the coelenterazine synthesized by the methods of the present disclosure.

### DETAILED DESCRIPTION

Disclosed herein are synthesis methods for making coelenterazine, in high yield and with good purity. Also disclosed are articles including the coelenterazine and coelenterazine derivatives. Representative absorbent articles include disposable diapers and adult incontinence products.

Chemiluminescence results from a chemical reaction that produces light and therefore provides a lighted indication of moisture that can be seen in low light and/or in the absence of light, and through clothes. Furthermore, chemiluminescence requires no external excitation light, as is required for photoluminescent (e.g., fluorescence) indicators. Accordingly, by generating light upon contact with an aqueous system (e.g., urine), the disclosed embodiments greatly enhance the ability of absorbent articles to indicate the occurrence of an insult in darkened conditions (e.g., at night). Moreover, by generating light that can be seen through clothing, a caregiver may be able to ascertain the occurrence of an insult without having to move or disturb the infant or adult wearer of such an absorbent article, such as during sleep.

The articles provided herein may provide the distinct advantages of insult indication at night and through clothes, which may reduce or even eliminate the need for caregivers to disturb the sleep (e.g., by pulling down clothes and/or shining a light) of one wearing an absorbent article in order to test for an insult. Further, because light (e.g., visible light) is produced by the chemiluminescent systems disclosed herein, there is no need to expose the absorbent article and/or the wearer to UV light in order to determine whether an insult has occurred, allowing health concerns associated with UV radiation to be avoided. Examples of articles including chemiluminescent materials are described, for example, in U.S. Application No. 14/516,255, the disclosure of which is herein incorporated in its entirety.

The articles of the present disclosure provide improved ease with which an insult can be detected, which allows the caregiver to check for an insult more frequently, due to the reduced interruption required. More frequent checks may allow an insult to be detected sooner and the absorbent article changed soon after the insult, thereby reducing the amount of time the insult contacts the wearer's skin, as well as reducing the possibility of fluid from multiple insults contacting the wearer's skin. The skin health and general comfort of the wearer are improved when the length of time that fluid is in contact with the skin is reduced. In some embodiments, the component amounts/ratios can be calibrated so that instead of seeing a peak and then a fade after each of a sequence of insults, once there is sufficient water present in the absorbent article, the luminescence can be maintained at a relatively steady intensity (e.g., the luminescence can vary less than about 30%, less than about 20%, less than about 10%, less than 30%, less than 20%, or less than 10%,) over a period of time (e.g., about 24 hours, about 12 hours, about 6 hours, about 3 hours, about 2 hours, about 1 hour, about 30 minutes, about 15 minutes, 24 hours, 12 hours, 6 hours, 3 hours, 2 hours, 1 hour, 30 minutes, or 15 minutes). As used herein, the word "about" as it relates to a quantity indicates a number within range of minor variation above or below the stated reference number. For example, "about" can refer to a number within a range of 10%, 9%, 8%, 7%, 6%, 5%, 4%, 3%, 2%, or 1% above or below the indicated reference number. In some embodiments, "about" refers to a number within a range of 5% above or below the indicated reference number. In some embodiments, "about" refers to a number within a range of 10% above or below the indicated reference number. In some embodiments, "about" refers to a number within a range of 1% above or below the indicated reference number.

### Synthesis of Coelenterazine

In another aspect, the present disclosure features a method of making coelenterazine, including coupling 4-(5-amino-6-benzylpyrazin-2-yl)phenol (coelenteramine) with 3-(4-(benzyloxy)phenyl)-2-oxopropanal to provide 8-benzyl-2-(4-(benzyloxy)benzyl)-6-(4-hydroxyphenyl)imidazo[1,2-α]pyrazin-3(7H)-one; and deprotecting the 8-benzyl-2-(4-(benzyloxy)benzyl)-6-(4-hydroxyphenyl)imidazo[1,2-α]pyrazin-3(7H)-one to provide 8-benzyl-2-(4-hydroxybenzyl)-6-(4-hydroxyphenyl)imidazo[1,2-α]pyrazin-3(7H)-one (coelenterazine).

The coelenteramine can be made via different routes, as outlined below. The methods can provide coelenterazine in good yield and at good purity.

### Coelenteramine synthesis

In some embodiments, the coelenteramine is made by first (a1) reacting 3-benzylpyrazin-2-amine (25) with N-bromosuccinimide to provide 3-benzyl-5-bromopyrazin-2-amine (2), or a salt thereof; or by (a2) reacting 3,5-dibromopyrazin-2-amine and (bromomethyl)benzene in the presence of zinc, iodine, and a palladium catalyst to provide the 3-benzyl-5-bromopyrazin-2-amine (2), or a salt thereof. In a step (b), the 3-benzyl-5-bromopyrazin-2-amine (2) is then reacted in two sequential steps to provide 4-(5-amino-6-benzylpyrazin-2-yl)phenol (7) (coelenteramine).

The reaction of (a1) 3-benzylpyrazin-2-amine (25) with N-bromosuccinimide can be carried out in an organic solvent, such as CHCl₃ (chloroform) at room temperature (e.g., about 22 °C to 23 °C, 22 °C to 23 °C, or 22 °C) at atmospheric pressure (*i.e.*, about 1 atm, or 1 atm). Once the reaction is complete, the reaction mixture can be washed with water, and 3-benzyl-5-bromopyrazin-2-amine (2) can be isolated by removing the organic solvent under reduced pressure. In some embodiments, (a1) provides 3-benzyl-5-bromopyrazin-2-amine (2) in a yield of about 60% to about 85 % (e.g., 60% to 85 %) relative to 3-benzylpyrazin-2-amine (25) and in a purity of at least about 85 % (e.g., a purity of about 85% to about 95%, a purity of about 85% to about 100%, a purity of 85% to 95%, or a purity of 85% to 100%). As used herein, it is understood that % yield refers to mole % yield. As used herein, the purity of a given compound (when accompanied by the % yield of the given compound) refers to the weight percent purity relative to the mass of the pure compound calculated based on mole % yield.

In some embodiments, in (a2), the palladium catalyst is bis(triphenylphosphine) palladium(II) dichloride. The palladium catalyst can be present in an amount of about 5 to about 10 mole percent (e.g., 5 to 10 mole percent) relative to 3,5-dibromopyrazin-2-amine. In some embodiments, (a2) includes about 1:2 to about 1:3 molar equivalent (e.g., 1:2 to 1:3 molar equivalent) of 3,5-dibromopyrazin-2-amine to (bromomethyl)benzene. (a2) can provide 3-benzyl-5-bromopyrazin-2-amine (2) in a yield of about 55% to about 75% (e.g., 55% to 75%) relative to the 3,5-dibromopyrazin-2-amine at a purity of about 80% to about 95% (80% to 95%). In some embodiments, (a2) includes reacting 3,5-dibromopyrazin-2-amine and (bromomethyl)benzene for a duration of about 18 hours to about 30 hours (e.g., 18 hours to 30 hours). The reaction of 3,5-dibromopyrazin-2-amine and the (bromomethyl)benzene can occur at a temperature of about 25 to about 40 °C (e.g., 25 to 40 °C) at a pressure of about 1 atm (e.g., 1 atm).

In some embodiments, step (b) includes a first step of reacting the 3-benzyl-5-bromopyrazin-2-amine (2) with 4-methoxyphenyl boronic acid (4) in the presence of a palladium catalyst to provide 3-benzyl-5-(4-methoxyphenyl)pyrazin-2-amine (5), or salt thereof. The palladium catalyst can be a palladium (0) catalyst, for example, tetrakis(triphenylphosphine)palladium(0). The palladium catalyst can be present in an amount of about 5 to about 10 percent by weight (e.g., 5 to 10 percent by weight) relative to the 3-benzyl-5-bromopyrazin-2-amine (2). The first step in (b) can include about 1:1 to about 1:1.3 molar equivalent of the 3-benzyl-5-bromopyrazin-2-amine (2) to 4-methoxyphenyl boronic acid (4). In some embodiments, the 3-benzyl-5-bromopyrazin-2-amine (2) and the 4-methoxyphenyl boronic acid (4) are reacted together for a duration of about 120 minutes to about 300 minutes (e.g., 120 minutes to 300 minutes). The 3-benzyl-5-bromopyrazin-2-amine (2) and the 4-methoxyphenyl boronic acid (4) can be reacted together at a temperature of about 60 °C to about 90 °C (e.g., 60 °C to 90 °C) and at atmospheric pressure (*i.e.*, a pressure of about 1 atm, or 1 atm). The first step in (b) can provide the 3-benzyl-5-(4-methoxyphenyl)pyrazin-2-amine (5), or salt thereof, in a yield of about 60% to about 85% (e.g., 60% to 85%) relative to the 3-benzyl-5-bromopyrazin-2-amine (2) of the product at a purity of about 80 to about 95% (*i.e.*, a yield of 60% to 85% of the 80%-95% pure product). As used here, yield at a given purity range refers to the yield of the product having the described purity range.

Alternatively, in some embodiments, (b) includes a first step of reacting the 3-benzyl-5-bromopyrazin-2-amine (2) with (4-methoxyphenyl)boronic acid in the presence of a palladium catalyst (e.g., a palladium (II) catalyst, such as bis(benzonitrile) palladium(II) dichloride) to provide 3-benzyl-5-(4-methoxyphenyl)pyrazin-2-amine (5) or a salt thereof. The palladium (II) catalyst can be present in an amount of about 5 to about 10 (e.g., 5 to 10) mole percent relative to the 3-benzyl-5-bromopyrazin-2-amine (2). The reaction mixture can further include 1,4-bis(diphenylphosphino)butane, for example, in an amount of about 5 to about 10 (e.g., 5 to 10) mole percent relative to the 3-benzyl-5-bromopyrazin-2-amine (2). In some embodiments, the reaction mixture further includes toluene, aqueous sodium carbonate, and ethanol. The 3-benzyl-5-bromopyrazin-2-amine (2) can be reacted with the (4-methoxyphenyl)boronic acid for a duration of about 200 minutes to about 350 minutes (e.g., 200 to 350 minutes), and/or at a temperature of about 80 to about 110 °C (e.g., 80 to 100 °C) and at a pressure of about 1 atm (e.g., 1 atm). When the palladium (II) catalyst is used, 3-benzyl-5-(4-methoxyphenyl)pyrazin-2-amine (5) can be provided in a yield of about 65% to about 85% (e.g., 65% to 85%) relative to the 3-benzyl-5-bromopyrazin-2-amine (2). The 3-benzyl-5-(4-methoxyphenyl)pyrazin-2-amine (5) can be isolated by diluting the reaction mixture with water and extracting with ethyl acetate. The ethyl acetate extract can be removed from the extract under reduced pressure to provide 3-benzyl-5-(4-methoxyphenyl)pyrazin-2-amine (5).

The 3-benzyl-5-(4-methoxyphenyl)pyrazin-2-amine (5), or salt thereof, regardless of whether obtained with a palladium (0) or palladium (II) catalyst, can be isolated by precipitation of the 3-benzyl-5-(4-methoxyphenyl)pyrazin-2-amine (5) as a hydrochloride salt. Precipitation is advantageous as it can eliminate column chromatography, which can be laborious, costly, and time-consuming; and/or can also increase yield relative to column chromatography. In some embodiments, isolating the 3-benzyl-5-(4-methoxyphenyl)pyrazin-2-amine (5) does not include chromatography (e.g., liquid chromatography), does not include recrystallization, or does not include chromatography and recrystallization. In some embodiments, the reaction mixture from the reaction of 3-benzyl-5-bromopyrazin-2-amine (2) with (4-methoxyphenyl)boronic acid is diluted with aqueous sodium chloride solution (e.g., an about 20% (e.g., 20%) by weight sodium chloride solution) and extracted with ethyl acetate. The ethyl acetate extract can then be treated with HCl aqueous solution (e.g., an about 3N (e.g., 3N) HCl_{(aq)} solution) and the 3-benzyl-5-(4-methoxyphenyl)pyrazin-2-amine hydrochloride salt product (5) can be isolated by filtration with a purity of about 75% to about 95% (e.g., 75% to 95%).

In some embodiments, (b) further includes a second step of deprotecting the 3-benzyl-5-(4-methoxyphenyl)pyrazin-2-amine (5), or salt thereof, to provide the 4-(5-amino-6-benzylpyrazin-2-yl)phenol (7) (coelenteramine). The deprotection can include subjecting the 3-benzyl-5-(4-methoxyphenyl)pyrazin-2-amine (5) to pyridinium chloride. Treatment with pyridinium chloride can proceed at an elevated temperature of about 180 to about 220 °C (e.g., 180 to 220 °C, or 200 °C) at atmospheric pressure. In some embodiments, the elevated temperature can cause the pyridinium chloride to separate from the reaction mixture by evaporation from the reaction mixture. Alternatively, in certain embodiments, deprotection can include subjecting the 3-benzyl-5-(4-methoxyphenyl)pyrazin-2-amine (5) to sodium hydride and ethanethiol in N,N'-dimethyl-formamide (DMF). When treated with sodium hydride and ethanethiol in DMF, the reaction mixture can be at a temperature of about 90 °C to about 120 °C (e.g., about 100 °C to 110 °C, 90 °C to 120 °C, or 100 °C to 110 °C) and/or for a period of about 15 minutes to about 5 hours (e.g., about 30 minutes to about 2 hours, about 30 minutes to about 1 hour, 30 minutes to 2 hours, 30 minutes to 1 hour, or 30 minutes). Once the reaction is complete, the mixture can be cooled to about 30 °C to about 50 °C (e.g., about 35 °C to about 45 °C, about 40 °C, 35 °C to 45 °C, or 40 °C), extracted with water and an organic solvent (e.g., ethyl acetate). The organic layer can then be separated, refluxed, and then cooled to about 5 °C to 20 °C (e.g., about 10 °C to 15 °C, 5 °C to 20 °C, or 10 °C to 15 °C), the 4-(5-amino-6-benzylpyrazin-2-yl)phenol (7) (coelenteramine) can be isolated by filtration. In both deprotection methods, the coelenteramine can be optionally purified by washing with an aqueous sodium hydroxide/dioxane solution, stirring with activated charcoal/silica, filtration, followed by precipitation by acidification of the filtrate, and isolation of the precipitated product by filtration. The deprotection can provide 4-(5-amino-6-benzylpyrazin-2-yl)phenol (7) (coelenteramine) in a yield of about 90% to about 100% (e.g., 90% to 100%, at least about 95%, at least 95%, at least about 98%, at least 98%, at least about 99%, or at least 99%) at a purity of about 85% to about 100% (e.g., 85 % to 100 %, about 90%, or 90%) relative to 3-benzyl-5-(4-methoxyphenyl)pyrazin-2-amine (5).

In some embodiments, coelenteramine is synthesized by (a) reacting 3,5-dibromopyrazin-2-amine and (bromomethyl)benzene in the presence of zinc, iodine, and a first palladium catalyst to provide 3-benzyl-5-bromopyrazin-2-amine (2); (b) reacting 3-benzyl-5-bromopyrazin-2-amine (2) with 4-methoxyphenyl boronic acid (4) in the presence of a palladium catalyst in a first step to provide a hydrochloride salt of 3-benzyl-5-(4-methoxyphenyl)pyrazin-2-amine (5), and deprotecting the hydrochloride salt of 3-benzyl-5-(4-methoxyphenyl)pyrazin-2-amine (5) in a second step to provide 4-(5-amino-6-benzylpyrazin-2-yl)phenol (7) (coelenteramine).

In some embodiments, coelenteramine is synthesized by reacting pyrazin-2-amine with benzyl chloride to provide 3-benzylpyrazin-2-amine; reacting 3-benzylpyrazin-2-amine (25) with N-bromosuccinimide to provide 3-benzyl-5-bromopyrazin-2-amine (2); and reacting the 3-benzyl-5-bromopyrazin-2-amine (2) in two sequential steps to provide 4-(5-amino-6-benzylpyrazin-2-yl)phenol (7) (coelenteramine). The two sequential steps for providing coelenteramine from 3-benzyl-5-bromopyrazin-2-amine (2) can include a first step of reacting the 3-benzyl-5-bromopyrazin-2-amine (2) with silyl-protected 4-bromophenol in the presence of magnesium and a palladium catalyst to provide silyl-protected 4-(5-amino-6-benzylpyrazin-2-yl)phenol. In some embodiments, the palladium catalyst is tetrakis(triphenylphosphine)palladium(0), which can be present in an amount of 1 percent or more (e.g., 2 percent or more, 3 percent or more, 4 percent or more, 5 percent or more, 6 percent or more, 7 percent or more, 8 percent or more, or 9 percent or more) and/or 10 percent or less (e.g., 9 percent or less, 8 percent or less, 7 percent or less, 6 percent or less, 5 percent or less, 4 percent or less, 3 percent or less, or 2 percent or less) by weight relative to the 3-benzyl-5-bromopyrazin-2-amine (2). For example, for every 100 grams of 3-benzyl-5-bromopyrazin-2-amine (2), 1 to 10 grams (e.g., 1 gram, 2 grams, 3 grams, or 5 grams) of palladium catalyst can be used. As an example, for every 100 grams of 3-benzyl-5-bromopyrazin-2-amine (2), 1 gram of palladium catalyst can be used. The two sequential steps can include a second step of deprotecting the silyl-protected 4-(5-amino-6-benzylpyrazin-2-yl)phenol to provide the 4-(5-amino-6-benzylpyrazin-2-yl)phenol (7), for example by subjecting the silyl-protected 4-(5-amino-6-benzylpyrazin-2-yl)phenol to aqueous HCl. This synthesis procedure reduces or eliminates the use of: n-butyl lithium-in the first step of the synthesis, by replacing the n-butyl lithium reaction in toluene with benzyl chloride and THF (tetrahydrofuran). Thus, this synthesis presents the ability to scale up the reaction chemistry and can reduce the cost of the synthesis. In addition, the changes can improve the overall safety of the chemistry by replacing highly reactive materials with more stable materials. The omission of boronic acid compounds in the synthesis can greatly reduce the amount of expensive palladium catalyst in the reaction.

In some embodiments, coelenteramine is synthesized by reacting pyrazin-2-amine (24) with benzyl chloride to provide 3-benzylpyrazin-2-amine (25) (e.g., under Grignard conditions, such as by first providing a solution of magnesium, iodine, and ethyl bromide in a solvent before reacting pyrazin-2-amine (24) with benzyl chloride to provide 3-benzylpyrazin-2-amine (25); reacting 3-benzylpyrazin-2-amine (25) with N-bromosuccinimide to provide 3-benzyl-5-bromopyrazin-2-amine (2); reacting 3-benzyl-5-bromopyrazin-2-amine (2) with 4-methoxyphenyl boronic acid (4) in the presence of a palladium catalyst in a first step to provide a hydrochloride salt of 3-benzyl-5-(4-methoxyphenyl)pyrazin-2-amine (5), and deprotecting the hydrochloride salt of 3-benzyl-5-(4-methoxyphenyl)pyrazin-2-amine (5) in a second step to provide 4-(5-amino-6-benzylpyrazin-2-yl)phenol (7) (coelenteramine). In some embodiments, the deprotection of 3-benzyl-5-(4-methoxyphenyl)pyrazin-2-amine (5) includes exposing the 3-benzyl-5-(4-methoxyphenyl)pyrazin-2-amine (5) to an acidic environment, such as HBr in acetic acid, to provide 4-(5-amino-6-benzylpyrazin-2-yl)phenol (7) (coelenteramine).

The reaction of 3-benzylpyrazin-2-amine (25) with N-bromosuccinimide can be carried out in an organic solvent, such as CHCl₃ (chloroform) at room temperature (e.g., about 22 °C to 23 °C, 22 °C to 23 °C, or 22 °C) at atmospheric pressure (*i.e.*, about 1 atm, or 1 atm). Once the reaction is complete, the reaction mixture can be washed with water, and/or an aqueous acid (e.g., HCl_{(aq)}), and 3-benzyl-5-bromopyrazin-2-amine (2) can be isolated by removing the organic solvent under reduced pressure. In some embodiments, 3-benzyl-5-bromopyrazin-2-amine (2) is provided in a yield of about 60% to about 85 % (e.g., 70-75 %) relative to 3-benzylpyrazin-2-amine (25) and in a purity of at least about 85 % (e.g., a purity of about 85% to about 95%, a purity of about 85% to about 100%, a purity of 85% to 95%, a purity of 90-95%, or a purity of 85% to 100%).

In some embodiments, the 3-benzyl-5-bromopyrazin-2-amine (2) is reacted with 4-methoxyphenyl boronic acid (4) in the presence of a palladium catalyst to provide 3-benzyl-5-(4-methoxyphenyl)pyrazin-2-amine (5), or salt thereof. The palladium catalyst can be a palladium (0) catalyst, for example, tetrakis(triphenylphosphine)palladium(0), or a palladium (II) catalyst, such as bis(benzonitrile) palladium(II) dichloride. When a palladium (II) catalyst is used, the reaction mixture can further include 1,4-bis(diphenylphosphino)butane, for example, in an amount of about 5 to about 10 (e.g., 5 to 10) mole percent relative to the 3-benzyl-5-bromopyrazin-2-amine (2). The palladium catalyst can be present in an amount of about 5 to about 10 percent by weight (e.g., 5 to 10 percent by weight) relative to the 3-benzyl-5-bromopyrazin-2-amine (2). The first step in (b) can include about 1:1 to about 1:1.3 molar equivalent of the 3-benzyl-5-bromopyrazin-2-amine (2) to 4-methoxyphenyl boronic acid (4). In some embodiments, the 3-benzyl-5-bromopyrazin-2-amine (2) and the 4-methoxyphenyl boronic acid (4) are reacted together for a duration of about 120 minutes to about 300 minutes (e.g., 120 minutes to 300 minutes). The reaction solvent can include 1,4-dioxane and/or water. In some embodiments, the reaction mixture further includes potassium carbonate in an amount of about 75 to about 85 mole percent relative to the 3-benzyl-5-bromopyrazin-2-amine (2). The 3-benzyl-5-bromopyrazin-2-amine (2) and the 4-methoxyphenyl boronic acid (4) can be reacted together for a duration of about 12 to about 36 hours (*e.g.*, about 20 to 24 hours, about 12 to about 24 hours, or about 15 to 24 hours), and/or at a temperature of about 80 to about 110 °C (e.g., 80 to 100 °C, or 80 to 85 °C), and/or at atmospheric pressure (*i.e.*, a pressure of about 1 atm, or 1 atm). The 3-benzyl-5-(4-methoxyphenyl)pyrazin-2-amine (5), or salt thereof, can be provided in a yield of about 65% to about 95% (e.g., 85% to 90%, 80% to 95%, or 85% to 90%) relative to the 3-benzyl-5-bromopyrazin-2-amine (2), at a purity of for example, 90% to 95% (*e.g.*, 92% to 95%). The 3-benzyl-5-(4-methoxyphenyl)pyrazin-2-amine (5) can be isolated by diluting the reaction mixture with water and extracting with ethyl acetate. The ethyl acetate extract can be removed from the extract under reduced pressure to provide 3-benzyl-5-(4-methoxyphenyl)pyrazin-2-amine (5).

The 3-benzyl-5-(4-methoxyphenyl)pyrazin-2-amine (5), or salt thereof, regardless of whether obtained with a palladium (0) or palladium (II) catalyst, can be isolated by precipitation of the 3-benzyl-5-(4-methoxyphenyl)pyrazin-2-amine (5) as a hydrochloride salt. Precipitation is advantageous as it can eliminate column chromatography, which can be laborious, costly, and time-consuming; and/or can also increase yield relative to column chromatography. In some embodiments, isolating the 3-benzyl-5-(4-methoxyphenyl)pyrazin-2-amine (5) does not include chromatography (e.g., liquid chromatography), does not include recrystallization, or does not include chromatography and recrystallization. In some embodiments, the reaction mixture from the reaction of 3-benzyl-5-bromopyrazin-2-amine (2) with (4-methoxyphenyl)boronic acid is diluted with aqueous sodium chloride solution (*e.g.*, an about 20% (e.g., 20%) by weight sodium chloride solution) and extracted with ethyl acetate. The ethyl acetate extract can then be treated with HCl aqueous solution (e.g., an about 3N (e.g., 3N) HCl_{(aq)} solution) and the 3-benzyl-5-(4-methoxyphenyl)pyrazin-2-amine hydrochloride salt product (5) can be isolated by filtration with a purity of about 75% to about 95% (e.g., 75% to 95%).

The 3-benzyl-5-(4-methoxyphenyl)pyrazin-2-amine (5), or salt thereof, can then be deprotected to provide 4-(5-amino-6-benzylpyrazin-2-yl)phenol (7) (coelenteramine). In some embodiments, the deprotection includes exposing the 3-benzyl-5-(4-methoxyphenyl)pyrazin-2-amine (5), or salt thereof, to an acid (*e.g.*, HBr and acetic acid). For example, the HBr can have an HBr concentration of 48% in water, and the aqueous HBr can be mixed with acetic acid in a ratio of from about 1:2 (*e.g.*, from about 1:1.5, from about 1:1.25) to about 1:1 (*e.g.*, to about 1:1.25, or to about 1:1.5). The deprotection can occur at a temperature of from about 100 °C (*e.g.*, from about 105 °C, from about 110 °C, from about 115 °C) to about 120 °C (*e.g.*, to about 115 °C, to about 110 °C, to about 105 °C) for a duration of from about 5 hours (*e.g.*, from about 8 hours, from about 10 hours, from about 12 hours, or from about 14 hours) to about 18 hours (*e.g.*, to about 14 hours, to about 12 hours, to about 10 hours, or to 8 hours), for example, from 8 to 10 hours, at atmospheric pressure. After the deprotection reaction, the reaction mixture can be cooled, extracted with an organic solvent (such as ethyl acetate), and the organic solvent can be removed under reduced pressure. The residue can then be refluxed with a hydrocarbon (such as cyclohexane), filtered, isolated, and dried to provide 4-(5-amino-6-benzylpyrazin-2-yl)phenol (7) (coelenteramine). The 4-(5-amino-6-benzylpyrazin-2-yl)phenol (7) (coelenteramine) can be obtained in an yield of from about 70% (*e.g.*, from about 75%, from about 80%, or from 85%) to about 90 % (*e.g.*, to about 85%, to about 80%, or to about 75%), for example, from 75% to 80%, at a purity of from about 85% (*e.g.*, from about 87%, from about 90%, or from about 92%) to about 95% (e.g., to about 92 %, (*e.g.,* to about 90%, or to about 87%), for example, a purity of about 90%, relative to 3-benzyl-5-(4-methoxyphenyl)pyrazin-2-amine (5).

### Synthesis of 3-(4-(benzyloxy)phenyl)-2-oxopropanal

In some embodiments, the present disclosure features a method of making 3-(4-(benzyloxy)phenyl)-2-oxopropanal, including providing 1-(benzyloxy)-4-(chloromethyl)benzene, and reacting the 1-(benzyloxy)-4-(chloromethyl)benzene in two steps to provide 3-(4-(benzyloxy)phenyl)-2-oxopropanal. The method of making 3-(4-(benzyloxy)phenyl)-2-oxopropanal does not include more than one palladium-catalyzed reaction, from an initial starting material of 4-hydroxybenzaldehyde.

The method can include a first step of reacting the 1-(benzyloxy)-4-(chloromethyl)benzene with methyl 2,2-dimethoxyacetate under Grignard conditions (e.g., ethyl bromide, magnesium, and a catalytic amount of iodine) to provide 3-(4-(benzyloxy)phenyl)-1,1-dimethoxypropan-2-one. The 3-(4-(benzyloxy)phenyl)-1,1-dimethoxypropan-2-one can be purified by silica column chromatography.

The method can include a second step of reacting the 3-(4-(benzyloxy)phenyl)-1,1-dimethoxypropan-2-one with acid (*e.g*., aqueous acid, such as 10% aqueous HCl)) to provide the 3-(4-(benzyloxy)phenyl)-2-oxopropanal. The 3-(4-(benzyloxy)phenyl)-2-oxopropanal can be isolated in a yield of 60 to 75% (*e.g.*, 65% to 70%) at a purity of 85 to 95% (*e.g.*, 90%) relative to 1-(benzyloxy)-4-(chloromethyl)benzene.

In some embodiments, the intermediate 1-(benzyloxy)-4-(chloromethyl)benzene can be made, for example, by the following procedure. A mixture of 4-hydroxybenzaldehyde (8), benzyl chloride, and anhydrous potassium carbonate in N,N-dimethyl formamide can be formed, heated to a temperature of about 40-80 °C for a duration of from, for example, 5 hours to 3 days, under atmospheric pressure. After reaction completion, the mixture can be cooled to room temperature, charged with water, and centrifuged or filtered to isolate the resulting 4-(benzyloxy)benzaldehyde.

The 4-(benzyloxy)benzaldehyde can then be reduced with sodium borohydride to provide (4-(benzyloxy)phenyl)methanol. Briefly, the sodium borohydride can be added at a temperature of about 45 to 50 °C dropwise to a solution of 4-(benzyloxy)benzaldehyde in methanol. The reaction mixture can then be cooled (*e.g.*, to about 15 °C), acidified (*e.g.*, with acetic acid), stirred with water, and the product can be isolated by filtration. The resultant product can be heated with an organic solvent (*e.g.*, n-hexane), filtered, and dried to provide (4-(benzyloxy)phenyl)methanol.

The (4-(benzyloxy)phenyl)methanol can then be reacted with thionyl chloride to provide 1-(benzyloxy)-4-(chloromethyl)benzene (11). For example, a mixture of (4-(benzyloxy)phenyl)methanol in dichloromethane and N,N'-dimethylformamide can be formed, to which thionyl chloride can be added slowly at a temperature of from about 30 to about 35 °C. After stirring the reaction for a duration of from about 30 minutes to about 2 hours, the solvent can be removed, and the residue can be extracted with water and an organic solvent, such as ethyl acetate. After separating the organic layer, the pH of the layer can be adjusted to about 8 to 9 with an aqueous base, such as a soda ash aqueous solution, then the organic layer can be separated again, washed with a sodium chloride aqueous solution, and the organic layer is then separated and concentrated under reduced pressure. The residue can then be washed with an organic solvent, such as n-hexane, and the product can be isolated by filtration and dried to obtain the intermediate 1-(benzyloxy)-4-(chloromethyl)benzene.

### Coelenterazine

In some embodiments, the coelenterazine is obtained by coupling 4-(5-amino-6-benzylpyrazin-2-yl)phenol (coelenteramine) with 3-(4-(benzyloxy)phenyl)-2-oxopropanal to provide 8-benzyl-2-(4-(benzyloxy)benzyl)-6-(4-hydroxyphenyl)imidazo[1,2-α]pyrazin-3(7*H*)-one; and deprotecting the 8-benzyl-2-(4-(benzyloxy)benzyl)-6-(4-hydroxyphenyl)imidazo[1,2-α]pyrazin-3(7*H*)-one to provide 8-benzyl-2-(4-hydroxybenzyl)-6-(4-hydroxyphenyl)imidazo[1,2-α]pyrazin-3(7*H*)-one (coelenterazine).

Coupling the 4-(5-amino-6-benzylpyrazin-2-yl)phenol (coelenteramine) with the 3-(4-(benzyloxy)phenyl)-2-oxopropanal can occur in a solvent mixture that includes dioxane, water, and HCl (*e.g.*, concentrated HCl, 36% HCl). The coupling reaction can be conducted at a temperature of 75 °C to 90 °C (e.g., 80 °C to 85 °C) for 12 to 36 hours (*e.g*., 24 hours) in an inert atmosphere.

In some embodiments, deprotecting the 8-benzyl-2-(4-(benzyloxy)benzyl)-6-(4-hydroxyphenyl)imidazo[1,2-α]pyrazin-3(7*H*)-one includes a first deprotection step of exposing the 8-benzyl-2-(4-(benzyloxy)benzyl)-6-(4-hydroxyphenyl)imidazo[1,2-α]pyrazin-3(7*H*)-one to acid, such as HCl (*e.g.*, concentrated HCl, 36% HCl). The reaction mixture can include an organic solvent, such as dioxane. The acid can be present in an amount equal to or in excess of the volume of the solvent mixture in the preceding coupling step. An intermediate deprotected product can be obtained, for example, by filtering the reaction mixture, collecting the solid residue, and drying the solid residue before proceeding to the following step. The first deprotection step can be conducted at a temperature of 25 °C to 40 °C (*e.g*., 30 to 35 °C), for a duration of from 30 minutes (*e.g.*, from 1 hour) to 2 hours (*e.g*., to 1.5 hours). The intermediate deprotected product (*i.e.*, the solid residue) can be washed with an organic solvent (*e.g*., toluene) before filtering and drying.

Deprotecting the 8-benzyl-2-(4-(benzyloxy)benzyl)-6-(4-hydroxyphenyl)imidazo[1,2-α]pyrazin-3(7*H*)-one can include a second deprotection step of exposing the dried intermediate deprotected product to acid, such as HCl (e.g., concentrated HCl, 36% HCl). The reaction mixture can include an organic solvent, such as dioxane. The second deprotection step can include heating the intermediate deprotected product in HCl and the organic solvent to a first temperature of about 50 °C to 75 °C (e.g., from 60 °C to 70 °C, from 55 °C to 65 °C, from 60 °C to 62 °C) for a duration of 6 to 24 hours, then at a second higher temperature (e.g., higher than the first temperature by 5 °C to 15 °C, by 5 °C to 10 °C, by 10 °C to 15 °C) to provide coelenterazine. To isolate the coelenterazine, the HCl and organic solvent can be removed under reduced pressure to provide a residue, the residue can be washed with organic solvents such as ethyl acetate, then dichloromethane, and hexane. The residue can be dried under reduced pressure, at room temperature or up to a temperature of about 50 °C (*e.g.*, 40-45 °C) to provide the final coelenterazine. The coelenterazine can be obtained in a yield of 70% or more (*e.g.*, 70 % to 95 %, 70 % to 85 %, 80% to 95%, 80% to 85%, or 80%) at a purity of from 55% to 70% (*e.g.*, 55% to 65%, or 60% to 65%) relative to 4-(5-amino-6-benzylpyrazin-2-yl)phenol.

The 3-(4-(benzyloxy)phenyl)-2-oxopropanal and the coelenteramine used in the coupling reaction to provide coelenterazine can be made by the methods describe above.

### Representative syntheses

In some embodiments, the coelenterazine of the present disclosure can be made according to Schemes G, H, and I, below. Scheme G illustrates the synthesis of coelenteramine, Scheme H illustrates the synthesis of 3-(4-(benzyloxy)phenyl)-2-oxopropanal, and Scheme F illustrates the coupling of coelenteramine and 3-(4-(benzyloxy)phenyl)-2-oxopropanal, and subsequent deprotection of the intermediate product 8-benzyl-2-(4-(benzyloxy)benzyl)-6-(4-hydroxyphenyl)imidazo[1,2-a]pyrazin-3(7H)-one, to generate 8-benzyl-2-(4-hydroxybenzyl)-6-(4-hydroxyphenyl)imidazo[1,2-a]pyrazin-3(7H)-one, coelenterazine (16).

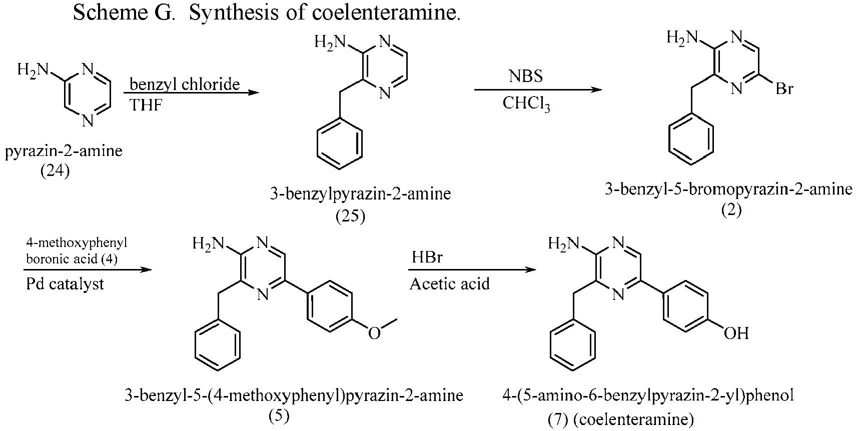

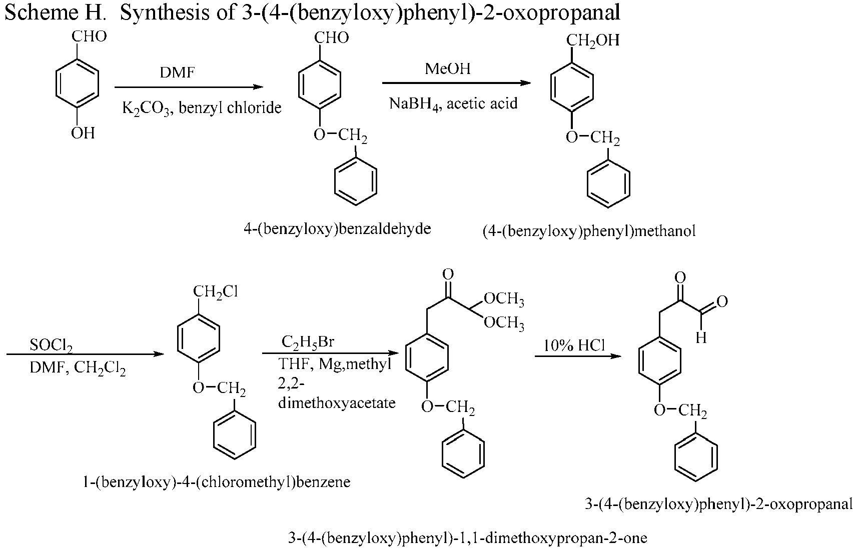

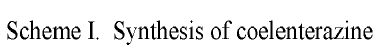

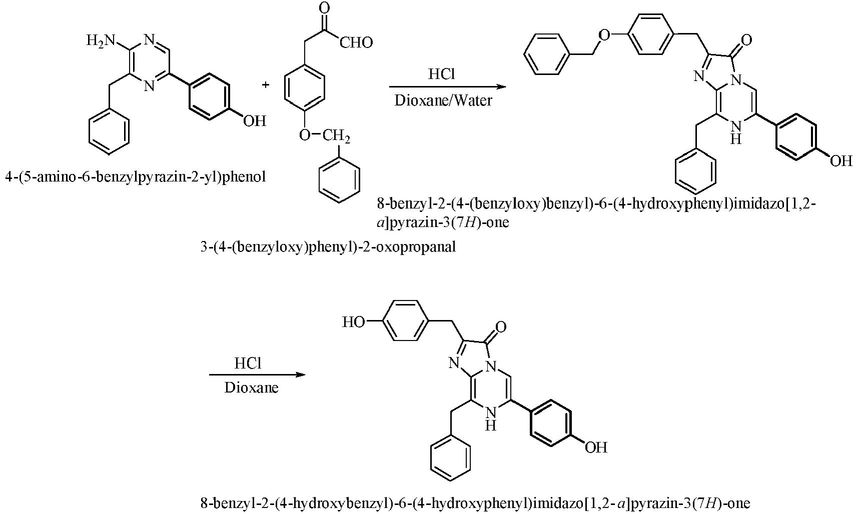

### Absorbent articles

The coelenterazine, or salt thereof, made according to the methods of the present disclosure can be incorporated into an absorbent article.

In some embodiments, the present disclosure features an absorbent article, including the coelenterazine, or salt thereof, synthesized by the methods of the present disclosure.

Materials and structural elements for absorbent articles that include one or more components of a chemiluminescent system are described, for example, in U.S. Provisional Application No. 62/692,502, titled "Chemiluminescent Wetness Indicator for Absorbent Products" and filed on June 29, 2018; US Provisional Application No. 62/753,024, titled "Chemiluminescent Wetness Indicator for Absorbent Products" and filed on October 30, 2018; and U.S. Application No. 16/457732, titled "Chemiluminescent Wetness Indicator for Absorbent Products" and filed on June 28, 2019.

### Chemiluminescent System

The chemiluminescent system is configured to produce light upon contact with an aqueous system. The aqueous system initiates the chemiluminescence reaction in order to produce light. As used herein, the term "aqueous system" refers to water or water-containing compositions. In the context of this disclosure, such water-containing compositions are generally in the form of body fluid, such as urine, menses, fecal matter, and so forth. The occurrence of the release of bodily fluid (or the fluid itself) is referred to herein as an "insult," or "liquid insult" or "fluid insult." Accordingly, the chemiluminescent systems of the present disclosure produce light upon insult of an article in which the system is incorporated.

In being configured to produce light upon contact with an aqueous system, the chemiluminescent system includes at least one compound or material that luminesces when contacted with an aqueous system. In one embodiment, water initiates the chemiluminescence.

In one embodiment, the chemiluminescent system includes two or more materials that luminesce when contacted with an aqueous system. In this embodiment, there are two or more materials that together do not luminesce without the presence of the aqueous system.

Representative chemiluminescent systems that include two or more materials include bioluminescent systems, such as a system that includes a luciferin and a luciferase.

Bioluminescence is light that is produced by a chemical reaction that occurs within the body or in the secretions of certain type of organisms. Bioluminescence involves the combination of two types of substances in a light-producing reaction: a luciferin and a luciferase. Luciferin is the compound that actually produces the light, for example, the luciferin can be coelenterazine. Luciferase is an enzyme that catalyzes the reaction. In some cases the luciferase is a protein known as a photoprotein, and the light making process requires a charged ion (e.g., a cation such as calcium) to activate the reaction. Photoprotein is a variant of luciferase in which factors required for light emission (including luciferin and oxygen) are bound together as one unit. Often, the bioluminescence process requires the presence of a substance such as oxygen or adenosine triphosphate (ATP) to initiate the oxidation reaction. The reaction rate for the luciferin is controlled by the luciferase or photoprotein. The luciferin-luciferase reaction can also create byproducts such as inactive oxyluciferin and water.

Luciferin and luciferase are generic names rather than specific materials. For example, the luciferin coelenterazine (natural form) is common in marine bioluminescence but variants can be chemically synthesized, and these various forms are collectively called luciferins. In another example, dinoflagellates (marine planktons) that obtain food through photosynthesis use a luciferin that resembles the chlorophyll structure.

The mechanism of light production through a chemical reaction differentiates bioluminescence from other optical phenomenon such as fluorescence or phosphorescence.

For example, fluorescent molecules do not emit their own light. They need an external photon source to excite the electrons to a higher energy state. On relaxation from the high energy state to their natural ground state, they release their acquired energy as a light source, but usually at a longer wavelength. Since the excitation and relaxation occurs simultaneously, fluorescent light is seen only when illuminated (excited).

The term phosphorescence technically refers to a special case of optically excited light emission where the relaxation from the excited state to ground state, unlike the fluorescence, is not immediate, and the photon emission persists for seconds to minutes after the original excitation.

The technical distinction between bioluminescence and fluorescence is sometimes blurred in a practical context but technically they are two distinct phenomena. In most cases, a bioluminescent can be an autofluorescent but the reverse is not true for a fluorescent; the latter still requires photon for excitation to emit light. In some cases a bioluminescent cnidarians or crustaceans or fish can contain a fluorescent protein like Green Fluorescent Protein (GFP) and the light emitted from the bioluminescent would act as photons to excite the GFP. The GFP in turn under relaxed state would emit a light of different wave length (most probably of higher wave length) than the wavelength of the bioluminescent light that it has received as photon. In this example, the GFP may be excited by a blue light emitted by the bioluminescent (wavelength about 470 nm, or 470 nm) but in turn would emit a green light under its relaxed state (wavelength of about 510 nm to about 520 nm, or 510 nm to 520 nm).

Bioluminescent systems can be incorporated into fluff pulp compositions, fiber matrices, or absorbent articles in any manner that produces the desired chemiluminescence.

In one embodiment, the fluff pulp composition or absorbent product comprises a luciferin selected from the group consisting of coelenterazine, dinoflagellate luciferin, bacterial luciferin, fungal luciferin, firefly luciferin, and vargulin. With regard to coelenterazine, there are many variants, any of which can be used in the fluff pulp composition.

Certain embodiments of coelenterazine consistent with this disclosure comprise one or more of native coelenterazine, methyl coelenterazine, coelenterazine 400a (2-2'(4-dehydroxy)) coelenterazine, coelenterazine e, coelenterazine f, coelenterazine h, coelenterazine i, coelenterazine n, coelenterazine cp, coelenterazine ip, coelenterazine fcp, and coelenterazine hep. As a further example, the coelenterazine may be one or more of native coelenterazine, coelenterazine 400a, methyl coelenterazine, coelenterazine f, coelenterazine cp, coelenterazine fcp, and coelenterazine hep. As yet a further example, the coelenterazine may be one or more of coelenterazine 400a, methyl coelenterazine and coelenterazine fcp. As yet a further example, the coelenterazine may be one or more of coelenterazine 400a, methyl coelenterazine, and coelenterazine hep. In yet another example, the coelenterazine may be may be one or more of coelenterazine 400a and coelenterazine hep.

In one embodiment, the luciferin has a concentration of 0.0005% to 0.002%, by weight of the fluff pulp. In one embodiment, the luciferin has a concentration of 0.0005% to 0.0015%, by weight of the fluff pulp. In one embodiment, the luciferin has a concentration of 0.0005% to 0.001%, by weight of the fluff pulp.

In some embodiments, the luciferin can be incorporated in any component of an absorbent article. For example, the luciferin (e.g., coelenterazine, or a coelenterazine salt of the present disclosure) can be incorporated into an absorbent article in an amount of from about 0.01 to about 100 mg (e.g., from about 0.01 to about 75 mg, from about 0.01 to about 50 mg, from about 0.01 to about 25 mg, from about 0.01 to about 10 mg, or from about 0.01 to about 5 mg), or 0.01 to 100 mg (e.g., from 0.01 to 75 mg, from 0.01 to 50 mg, from 0.01 to 25 mg, from 0.01 to 10 mg, or from 0.01 to 5 mg).

In one embodiment, the fluff pulp composition or absorbent product comprises luciferase selected from the group consisting of *Gaussia* luciferase (Gluc), *Renilla* luciferase (RLuc), dinoflagellate luciferase, firefly luciferase, fungal luciferase, bacterial luciferase, and vargula luciferase. Certain embodiments of the luciferase consistent with this disclosure comprise one or more of *Gaussia* luciferase, *Renilla* luciferase, dinoflagellate luciferase, and firefly luciferase. As a further example, the luciferase may be one or more of *Gaussia* luciferase, *Renilla* luciferase, dinoflagellate luciferase, and firefly luciferase. In yet a further example, the luciferase may be one or more of *Gaussia* luciferase and *Renilla* luciferase.

In one embodiment, the luciferase has a concentration of about 0.005% to about 0.04% (e.g., 0.005% to 0.04%) by weight of the fluff pulp. In one embodiment, the luciferase has a concentration of about 0.005% to about 0.02% (e.g., 0.005% to 0.02%) by weight of the fluff pulp. In one embodiment, the luciferase has a concentration of about 0.005% to about 0.01% (e.g., 0.005% to 0.01%) by weight of the fluff pulp.

In some embodiments, the luciferase can be incorporated in any component of an absorbent article. For example, the luciferase (e.g., GLuc) can be incorporated into an absorbent article in an amount of from about 0.2 mg to about 40 mg (e.g., from about 0.2 mg to about 30 mg, from about 0.2 mg to about 20 mg, from about 0.2 mg to about 15 mg, from about 0.2 mg to about 10 mg, from about 0.2 mg to about 5 mg, or from about 0.2 to about 2 mg); or from 0.2 mg to 40 mg (e.g., from 0.2 mg to 30 mg, from 0.2 mg to 20 mg, from 0.2 mg to 15 mg, from 0.2 mg to 10 mg, from 0.2 mg to 5 mg, or from 0.2 to 2 mg).

In one embodiment, the chemiluminescent system comprises coelenterazine as the luciferin and *Gaussia* or *Renilla* luciferase.

Representative luciferins include those of the coelenterazine family. Coelenterazine in its native form as well as its analogs have different luminescent characteristics due to variation in their structural moieties. Given structural variations within the coelenterazine family, some are good substrates for certain luciferases, whereas some are not. Below is a brief description of native coelenterazine and representative analogs.

Coelenterazine (native form) is a luminescent enzyme substrate for *Renilla (reniformis)* luciferase (Rluc). *Renilla* luciferase/coelenterazine has also been used as the bioluminescence donor in bioluminescence resonance transfer (BRET) studies.

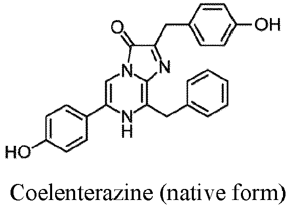

Coelenterazine 400a is a derivative of coelenterazine and is a good substrate for *Renilla* luciferase, but does not oxidize well with *Gaussia* luciferase (Gluc). It is the preferred substrate for BRET (bioluminescence resonance energy transfer) because its emission maximum of about 400 nm (e.g., 400 nm) has minimal interference with the GFP emission.

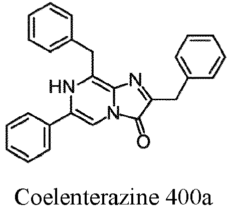

Fluorescence resonance energy transfer (FRET), BRET, resonance energy transfer (RET), and electronic energy transfer (EET) are mechanisms describing energy transfer between two light-sensitive molecules (chromophores) and can define the interference of a luminescent chemical with another luminescent chemical's energy transfer. A donor chromophore, initially in its electronic excited state, may transfer energy to an acceptor chromophore through nonradiative dipole-dipole coupling. The efficiency of this energy transfer is inversely proportional to the sixth power of the distance between donor and acceptor, making FRET extremely sensitive to small changes in distance. Measurements of FRET
efficiency can be used to determine if two fluorophores are within a certain distance of each other.

Such measurements are used as a research tool in fields including biology and chemistry.

Coelenterazine cp in a coelenterazine-aequorin complex generates luminescence intensity about 15 times (e.g., 15 times) higher than coelenterazine (native form).

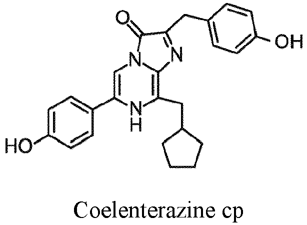

Coelenterazine f has about 20 times (e.g., 20 times) higher luminescence intensity (coelenterazine-apoaequorin complex) than the native form coelenterazine, while its emission maximum is about 8 nm (e.g., 8 nm) longer than that of the native form.

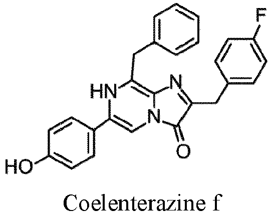

Coelenterazine fcp is an analog wherein the a-benzene structure in the coelenterazine moiety of coelenterazine f structure is replaced with a cyclic pentane (similar to coelenterazine cp). Coelenterazine fcp has luminescence intensity about 135 times (e.g., 135 times) greater than that of coelenterazine (native form).

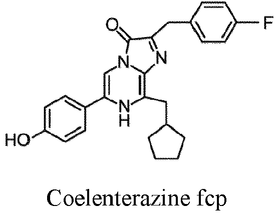

Coelenterazine fcp complexes with aequorin to form a coelenterazine fcp-apoaequorin complex, and, as a substrate for aequorin, has a relative luminescence intensity of about 135 times (e.g., 135 times) that of native coelenterazine. However, coelenterazine fcp is a poor substrate for *Renilla* luciferase.

Other representative analogs of coelenterazine, as a substrate for *Renilla* Luciferase enzyme, are coelenterazine e, h and n. While these three analogs are good to excellent substrates for *Renilla* luciferase, they are poor substrates for apoaequorin.

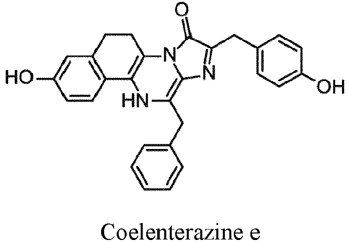

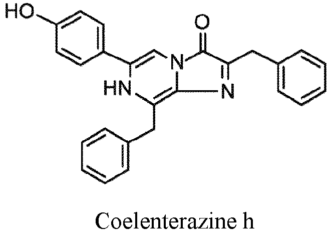

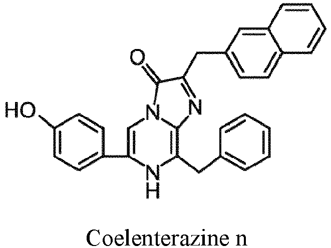

The luminescent properties of coelenterazine analogs vary. For example, certain analogs emit less light (as measured as lumens) but with higher luminescent intensity (lumens/steradian). Table A lists the luminescent properties of coelenterazine (native form) and its analogs with *Renilla* Luciferase. Luminescent intensity is reported as a % initial intensity. For example, an analog having an initial intensity of 900% is about 20 times (e.g., 20 times) intense as compared to the native coelenterazine with an initial intensity of about 45% (e.g., 45%).

**Table A: Luminescent Properties of Selected Coelenterazine Analogs with Renilla Luciferase**

| Analog | Aem(nm) | Total Light(%} | Initial Intensity(%) |
|---|---|---|---|
| native | 475 | 100 | 45 |
| e | 418,475 | 137 | 900 |
| f | 473 | 28 | 45 |
| h | 475 | 41 | 135 |
| n | 475 | 47 | 900 |
| cp | 470 | 23 | 135 |

Light is produced by the chemiluminescent system. The light is visually detectable by a caregiver in the dark and through clothing, and as such the light has a wavelength, intensity, and duration sufficient to provide the necessary indication. These spectral characteristics of the chemiluminescent system can be tailored based on the chemiluminescent compound or compounds. For example, in bioluminescent systems, the luciferin and luciferase can be selected to produce the desired light characteristics. Depending on the bioluminescent system used, different spectral characteristics can occur. In the presence of superoxide anions and/or peroxynitrile compounds, coelenterazine can also emit light independent of enzyme (luciferase) oxidation, a process known as autoluminescence.

The chemiluminescent system can be tailored to produce particular colors of light. As noted above in Table A, even within the coelenterazine family, the emission wavelength can range from about 400 nm (violet, e.g., 400 nm) to about 475 nm (blue with green tint, e.g., 475 nm).

With regard to duration, the duration of the light emitted may be controlled by the selection of the coelenterazine (luciferin), in native form versus its analogues, and the enzyme (Luciferase), for example *Gaussia* versus *Renilla.* The ratio and the concentration of luciferin and luciferase used may also modify the duration of light emission. To give an illustrative and non-limiting example, the luciferin analogue, coelenterazine e, has a total light of 130% and initial intensity of 900% over native coelenterazine. By judiciously selecting the concentration of coelenterazine e and *Renilla* luciferase, the duration of the light emitted can last as long as about 8 to about 10 hours (e.g., 8 to 10 hours).

In one embodiment, the light has a duration of about 0.5 to about 6 hours (e.g., 0.5 to 6 hours). In another embodiment, the light has a duration of about 1 to about 4 hours (e.g., 1 to 4 hours). In another embodiment, the light has a duration of about 2 to about 3 hours (e.g., 2 to 3 hours).

With regard to intensity, quantum efficiency of the chemiluminescence contributes to the intensity, depth, and hue of the color of the emission.

Quantum efficiency (QE) is the fraction of photon flux used to excite a luminescence chemical to elevate it to higher energy state. Quantum efficiency is one of the most important parameters used to evaluate the quality of a detector and is often called the "spectral response" to reflect its wavelength dependence. It is defined as the number of signal electrons created per incident photon. In some cases it can exceed 100% (i.e. when more than one electron is created per incident photon). If the spectral response exceeds 100%, then the intensity and depth of the color emitted is vivid, but depending on the status of the excited state of the primary electron, the duration of the emission will be determined (i.e., the higher the excited state, the more time it takes to return to the ground (normal) state).

Spectral responsivity is a similar measurement, but it has different units; the metric being the amperes or watts (i.e., how much current comes out of the device per incoming photon of a given energy and wavelength).

Both the quantum efficiency and the spectral responsivity are functions of the photons' wavelength. For example, in the case of the luciferin coelenterazine, between the native form and one of its analogs, coelenterazine e, the latter has not only high light intensity but emits 30% more light energy than the former, because the latter upon excitation by a given quanta (hv) of incident photon generates two electrons and the primary electron at wavelength 475 has the same emission intensity as native coelenterazine but with lumen intensity about 20 times (e.g., 20 times) greater than that of the native product. Accordingly, the light emitted by the excited coelenterazine analog would be twenty times brighter than the native form but with a total light energy of about 130% (e.g., 130%) will last longer than the native form.

The wavelength determines the color of the emitted light.

In one embodiment, the fluff pulp composition includes a luciferin and a luciferase. Such a fluff pulp has both elements of the chemiluminescent system required to luminesce upon contact with an aqueous system. However, in another embodiment, the fluff pulp composition includes at least one component selected from a luciferin and a luciferase. In such an embodiment, the fluff pulp composition may include only one of a luciferin and a luciferase. Such a fluff pulp composition may be incorporated into an absorbent article such that the other one of the luciferin and the luciferase may be disposed in a top sheet or other layer of the absorbent article, such that the two components are combined only when carried by a liquid insult (e.g., water from an aqueous system passing through the top sheet into the fluff pulp composition). In one embodiment the fluff pulp composition comprises a luciferin but not a luciferase. In one embodiment the fluff pulp composition comprises a luciferase but not a luciferin.

### Fluff Pulp

The fluff pulp of the fluff pulp composition can be formed from any pulp. In one embodiment, the fluff pulp is derived from a lignocellulosic fiber. In one embodiment, the fluff pulp is derived from a lignocellulosic fiber derived from wood. In one embodiment, the fluff pulp is derived from a lignocellulosic fiber derived from wood by chemical, mechanical, chemimechanical, or thermomechanical means. In one embodiment, the fluff pulp is derived from a cellulosic fiber derived from wood by chemical pulping. In one embodiment, the fluff pulp is derived from a cellulosic fiber derived from chemical pulping of wood either by alcohol pulping, organo-solve pulping, acid sulfite pulping, alkaline sulfite pulping, neutral sulfite pulping, alkaline peroxide pulping, Kraft pulping, Kraft-AQ pulping, polysulfide pulping, or polysulfide-AQ pulping.

In one embodiment, the fluff pulp is derived from a cellulosic fiber derived from chemical pulping of wood by further removing lignin from the said pulp either by alcohol pulping, organo-solve pulping, acid sulfite pulping, alkaline sulfite pulping, neutral sulfite pulping, alkaline peroxide pulping, Kraft pulping, Kraft-AQ pulping, polysulfide pulping, or polysulfide-AQ pulping for the preparation of absorbent articles (fluff pulp). In one embodiment, the fluff pulp is derived from a cellulosic fluff pulp derived from Kraft pulping. In one embodiment, the fluff pulp is derived from a cellulosic bleached fluff pulp derived from Kraft pulping. In one embodiment, the fluff pulp is derived from a cellulosic bleached fluff pulp derived from Kraft pulping of softwoods. In one embodiment, the fluff pulp is derived from a cellulosic bleached fluff pulp derived from Kraft pulping of Southern softwoods. In one embodiment, the fluff pulp is derived from a cellulosic bleached fluff pulp derived from Kraft pulping of Southern pine. In one embodiment, the fluff pulp is derived from a Southern softwood. In one embodiment, the fluff pulp is derived from Southern pine.

The fluff pulp composition can be produced from pulp in any form, such as a wet-laid sheet which is dried to achieve a moisture content ranging from about 6% to about 11% (e.g., 6% to 11%).

In another aspect, methods of preparing the fluff pulp composition are provided. The fluff pulp composition is prepared by incorporating at least one component of the chemiluminescent system into the fluff pulp.

This can be accomplished using various methods that allow the fluff pulp to be treated with one or more components of the chemiluminescent system. One challenge in the chemical treatment of fluff pulp is to maintain the chemicals in a state in which the intended chemiluminescent reaction is not prematurely triggered, for example, before the treated fluff pulp is incorporated into an absorbent article that is then subjected to a liquid insult. For a wet end application, the chemicals typically cannot be comingled with water and be applied together. Accordingly, in an illustrative example, either the luciferase or luciferin may be microencapsulated and introduced during the wet-laying process, with the non-encapsulated component applied to the sheet in a non-aqueous environment by standard methods such as coating, dipping, spraying, or printing (or combination thereof), prior to the air-laid operation during absorbent article manufacture. In another illustrative example, a two sheet system, one containing luciferase and the other containing luciferin, may be made and processed further before the air-laid operation during absorbent article manufacture. In yet other examples, one of the chemicals maybe added during the wet-laying process and the other during the subsequent processing of the pulp; or the two components may be added to the pulp during or prior to the air-laid process, such as by rinsing and/or spraying the pulp in fluffed form with non-aqueous solutions of one or both the respective components.

### Absorbent Articles

In one embodiment, the fluff pulp composition; the isolated composition including the coelenterazine, or salt thereof; and/or the coelenterazine, or salt thereof, can be incorporated into absorbent articles. Representative absorbent articles include child diapers, adult diapers, adult incontinence products, feminine hygiene products, absorbent underpads, and wound care dressing articles. For example, the fluff pulp composition and/or the coelenterazine, or salt thereof, can be incorporated into one or more absorbent layers or portions of an absorbent article.

In another aspect, an absorbent article is provided. In one embodiment, the absorbent article includes a top sheet that is liquid permeable, a back sheet that is liquid impermeable, fluff pulp disposed between the top sheet and the back sheet and/or a fluffless or near fluffless non-woven fabric matrix disposed between the top sheet and the back sheet, and a chemiluminescent system configured to produce light upon contact with an aqueous system.

The chemiluminescent system (e.g., a luciferin such as coelenterazine, or a salt thereof of the present disclosure, and a luciferase) of the absorbent article is as described herein. However, the chemiluminescent system need not be disposed, in whole or in part, within the fluff pulp. As discussed above, structural and fluid distribution functions may be provided, in some configurations, by synthetic fibers, leading to the development of absorbent cores containing both fluff pulp fibers and synthetic fibers, and even "fluff-less" absorbent cores containing no fluff pulp fibers. In some embodiments, the chemiluminescent system, or parts of the chemiluminescent system, can be independently integrated in the liquid permeable top sheet, the liquid impermeable back sheet, the SAP, or another structure in the absorbent article.

In one embodiment, the chemiluminescent system comprises a luciferin and a luciferase. In one embodiment the luciferin and the luciferase are both disposed within the fluff pulp. In another embodiment, one of the luciferin and the luciferase is disposed within the fluff pulp and the other is disposed in a different layer (e.g., top sheet or ADL) of the absorbent product such that the two components are combined only when at least one of the two components is carried by a liquid insult (e.g., passing through the top sheet or ADL into the fluff pulp composition). In one embodiment the fluff pulp comprises a luciferin but not a luciferase. In one embodiment the fluff pulp comprises a luciferase but not a luciferin.

In yet another embodiment, at least one component of the chemiluminescent system is disposed on (for example, printed onto) the inner surface of the backsheet.

In one embodiment, one of the luciferin and the luciferase is disposed within the fluff pulp and the other is associated with the top sheet or another structure within an article, and configured to travel into the fluff pulp upon exposure to a liquid insult.

In one embodiment, the absorbent article further comprises a pH buffer, as disclosed herein. In one embodiment, the pH buffer is disposed within the fluff pulp. As discussed above, structural and fluid distribution functions may be provided, in some configurations, by synthetic fibers, leading to the development of absorbent cores containing both fluff pulp fibers and synthetic fibers, and even "fluff-less" absorbent cores containing no fluff pulp fibers. In some embodiments, the chemiluminescent system, or parts of the chemiluminescent system, can be independently integrated in the liquid permeable top sheet, the liquid impermeable back sheet, the SAP, or another structure in the absorbent article.

In one embodiment, the absorbent article further comprises a photoluminescent compound, as disclosed herein. In one embodiment, the photoluminescent compound is disposed within the fluff pulp.

In one embodiment, the absorbent article further comprises a photoluminescent compound and a pH buffer, as disclosed herein. In one embodiment, the photoluminescent compound and the pH buffer are disposed within the fluff pulp.

In one embodiment, the pH buffer, the photoluminescent compound, the luciferin, and the luciferase are disposed within the fluff pulp.

In one embodiment, at least one of the pH buffer, the photoluminescent compound, the luciferin, and the luciferase are not disposed within the fluff pulp. In some embodiments, at least one of the pH buffer, the photoluminescent compound, the luciferin, and the luciferase can be independently incorporated into synthetic fibers, "fluff-less" absorbent cores containing no fluff pulp fibers, in the liquid permeable top sheet, the liquid impermeable back sheet, and/or the SAP of the absorbent article. In some embodiments, the pH buffer, the photoluminescent compound, the luciferin, and/or the luciferase can migrate to an absorbent core from a different structure of the article. In some embodiments, the pH buffer, the photoluminescent compound, the luciferin, and/or the luciferase can migrate from an absorbent core to a different structure of the article.

In one embodiment, the absorbent article further includes a superabsorbent polymer (SAP), such as incorporated in the absorbent core. In such an embodiment, at least one component of the chemiluminescent system may be disposed in the SAP, such that the chemiluminescence is generated upon the fluid from an insult traveling to the absorbent core.

In one embodiment the chemiluminescent system is contained entirely within an absorbent core of the absorbent article. As the absorbent core is almost always a multicomponent system there exist more than one approach to incorporate the chemiluminescent system into the absorbent core. For instance the fluff pulp fibers could be the carrier of the chemiluminescent system. Alternatively, the chemiluminescent system could be contained within superabsorbent particles incorporated into the absorbent article. In some embodiments, the absorbent core can include cellulose fibers, cellulose fiber derivatives (rayon, lyocell, etc.), nonwoven cellulose fibers and/or cellulose fiber derivatives, non-cellulose fibers, or any combination thereof.

Furthermore, if only a portion of the SAP particles or fibers contained the chemiluminescent system chemistry, a desired pattern, such as an aesthetically pleasing pattern, can be achieved.

The chemiluminescent system can be added to the fluff pulp fibers at the time of absorbent article manufacture or during an upstream process entirely separated from final product assembly. As noted above, for example, the chemiluminescent system may be sprayed onto or otherwise incorporated into a fluff pulp sheet prior to hammermilling.

In another aspect, methods of manufacturing absorbent articles are also disclosed.

Absorbent articles are manufactured according to general techniques known to those of skill in the art that allow the incorporation of the chemiluminescent system to be incorporated into the absorbent article in the manner disclosed herein.

The following example is intended to be illustrative, not limiting.

### EXAMPLES

### Example 6. Synthesis of Coelenterazine using 4-(5-amino-6-benzylpyrazin-2-yl)phenol and 3-(4-(benzyloxy)phenyl)-2-oxopropanal

### 4-(5-Amino-6-benzyl-pyrazin-2-yl) phenol (coelenteramine) 3-Benzylpyrazin-2-amine

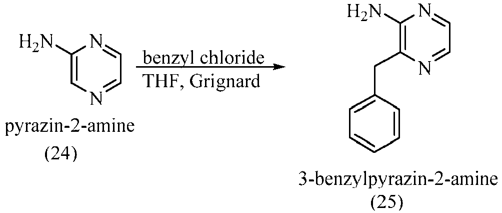

A 100 L round bottom flask was charged with 7.5 L of tetrahydrofuran (THF), 2.5 kg magnesium metal, 10g of iodine, and 200 ml ethyl bromide. The reaction mass was initiated, benzyl chloride and THF solution (10L benzyl chloride dissolved in 45L of THF) was slowly added at 10-20 °C over a period of 4 to 4.5 hours and thereafter maintained for 1 hour at 30-35 °C. Then 2-amino pyrazine solution (2.5kg 2-amino pyrazine dissolved in 25L THF at 30-35 °C in 1 to 1.5 hrs) was slowly added and the reaction mass was maintained for 5-6 hours at 30-35 °C. A thin layer chromatography (TLC) check was done, after compliance 10 L water was slowly added and the reaction mass was stirred for 20 minutes.

The reaction mass was then allowed to settle for 30 mins. The THF layer was separated and distilled out completely under vacuum below 70 °C. After completing the distillation, the reaction mass was then cooled to room temperature and charged with 20 L toluene and 5 L water and stirred for 10 minutes. The reaction mass was then allowed to settle for 20 minutes and the toluene layer was separated. The toluene layer was then charged with 3L HCl and allowed to settle for 10 minutes. Then the toluene layer was separated and kept aside. The acidic HCl layer was then adjusted to a pH of about 8-9 with 3 kg soda ash and maintained for 30 minutes. The organic layer was then separated to yield the desired mono alkylated product in 45-50% yield, at a purity 90-95%.

### 3-Benzyl-5-bromopyrazin-2-amine

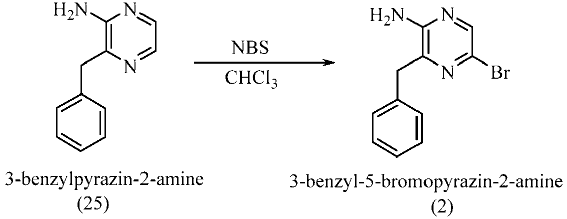

A 20 L round bottom flask was charged with chloroform (6 L) and 2-amino-3-benzyl pyrazine (1 Kg), and stirred the mixture at room temperature (22 °C). N-bromosuccinimide (800 grams) was added slowly over 1-1.5h. After the complete addition, the mixture was stirred for 30 minutes. Water (2 L) was added and stirred for 10 minutes followed by the separation of the chloroform layer. HCl (500ml) was added to the chloroform layer and the mixture was stirred for about 20 minutes. The product was filtered through Nutsche filter and was given a wash with chloroform (1L). The product was dried at 35-40 °C for 6-7 hrs. Yield 70-75%. Purity 90-95%.

### 3-Benzyl-5-(4-methoxyphenyl)pyrazin-2-amine

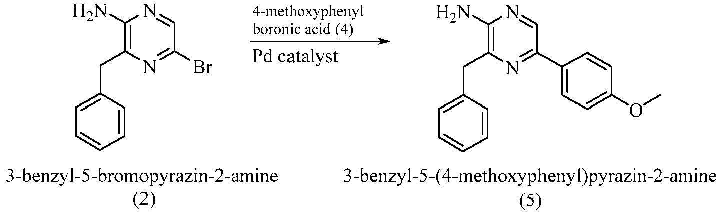

A 50 L flask was charged with 1, 4-dioxane (30 L) and 3-benzyl-5-bromopyrazin-2-amine (1 kg) at room temperature. Potassium carbonate (1.6 kg) was added followed by water (5 L). The reaction mixture was stirred for 10 minutes. 4-Methoxy phenyl boronic acid (600 grams) was added followed by palladium catalyst (40 grams). The reaction mixture was slowly heated up to 82°C and stirred at 80-82°C for 20-24h under nitrogen. The mixture was cooled to 40 °C and transferred to 100 L round bottomed flask, and charged with ethyl acetate (15 L) and water (15 L) at room temperature and stirred for 20 minutes. The organic layer was separated and concentrated under reduced pressure to yield the desired product. Yield 85-90%. Purity 92-95%.

### 4-(5-Amino-6-benzyl-pyrazin-2-yl) phenol

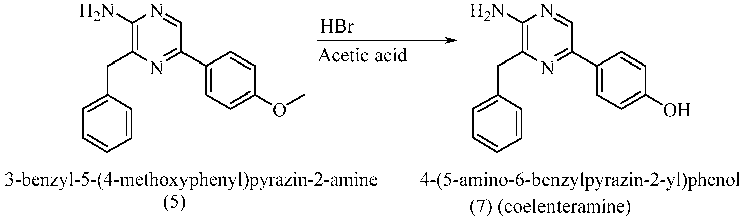

A 30 L round bottom flask was charged with 3-benzyl-5-(4-methoxyphenyl) pyrazin-2-amine (1 Kg), 4 L of 48% HBr and 7 L acetic acid. The reaction mixture was slowly heated to 110 ⁰C in an oil bath. The reaction temperature was maintained at 108-110 °C for 8-10 hours. The mixture was cooled to 40 ⁰C and charged with water (15L) and ethyl acetate (15 L) at 35-40 °C. The reaction mixture was stirred for 20 minutes. The pH of the mixture was adjusted to 4-4.5 using soda-ash and the mixture was stirred for 10 minutes.

The ethyl acetate layer was separated, and the aqueous layer was re-extracted with ethyl acetate (3 L x 2). The combined ethyl acetate extracts were adjusted to pH 7-7.5 with soda-ash and was concentrated under reduced pressure. The residue was first cooled to 40 °C and was then taken up with 4L of cyclohexane and refluxed. The compound was filtered through Nutsche filter and dried at 40-45 °C for 5-6 hrs. Yield: 75 - 80 % Purity: 90%.

### 3-(4-(benzyloxy)phenyl)-2-oxopropanal

### 4-(benzyloxy)benzaldehyde

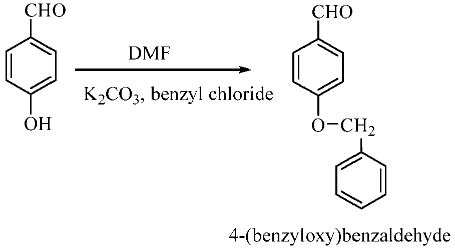

A 50L round bottom flask was charged with dimethylformamide (15 L), 4-hydroxybenzaldehyde (2.5 Kg) and the mixture was stirred for 20 minutes. To this mixture, potassium carbonate (4 Kg) was added and the reaction mass was stirred for 10 minutes after which benzyl chloride (2.5 L) was added slowly for approximately 15 minutes. The mixture was stirred for 20 minutes and was slowly heated to 40 °C followed by stirring at 45-45 °C for 6-7 hrs. The mixture was cooled to 30 °C and charged with water (25 L). The mixture was cooled further to 20 °C and stirred for 30 minutes. The product obtained was centrifuged and washed with water until the product became pH neutral. The product was then dried at 30-35 °C for 5-6 hrs. Yield : 70-75% Purity : 85-90%.

### (4-(benzyloxy)phenyl)methanol

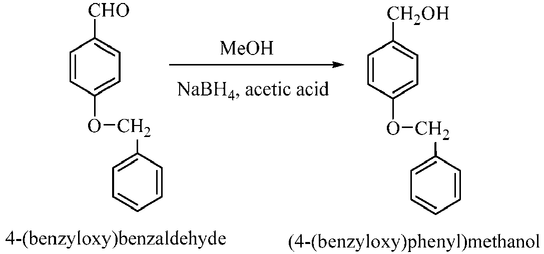

A 50L round bottom flask was charged with 4-(benzyloxy)benzaldehyde (4 Kg) and methanol (15L). To this mixture, sodium borohydride (0.5 Kg) was added drop-wise at 45-50 °C over 1 -1 ½ hrs. After the addition was completed, the mixture was stirred for 30 minutes. The reaction mixture was cooled to 15 °C and was charged slowly with aqueous acetic acid (500 mL acetic acid in 500mL water) followed by slow addition of water (25L). This mixture was then stirred for 30 minutes at 15-20 °C. The product was centrifuged and dried at 40-45 °C over 12-14 hrs. The resultant product was charged with n-hexane (10 L) and the reaction mixture was heated slowly to 50 °C. The mixture was stirred at 50 °C for 1 hr after which it was cooled to 40 °C. The product, (4-benzyloxy)phenyl)methanol was filtered through a Nutsche filter and it was then dried at 40-45 °C over 12 hrs. Yield : 85-90% Purity : 90-95%.

### 1-(benzyloxy)-4-(chloromethyl)benzene

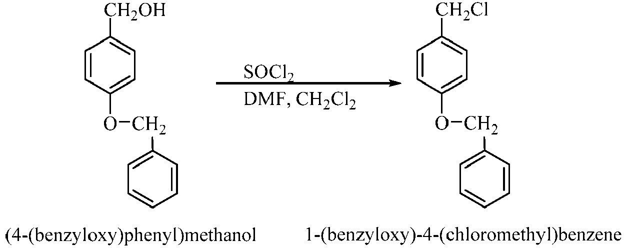

A 50L round bottom flask was charged with dichloromethane (20L) and (4-(benzyloxy)phenyl)methanol (3.8 Kg) at room temperature. The mixture was stirred for 20 minutes and was charged with dimethylformamide (500mL) followed by stirring for another 10 minutes. To this mixture, thionyl chloride (2L) was added slowly at 30-35 °C over 1 -1 ½ hrs. The mixture was stirred at room temperature for 1 -1 ½ hrs. 50 % of the dichloromethane was distilled normally and the remaining 50 % was removed by distillation under reduced pressure. The residue was charged with water (5L) and ethyl acetate (20L) and this mixture was stirred for 10 minutes.

The ethyl acetate layer was separated and this layer was washed with a soda-ash solution ( 1Kg soda-ash in 4L water) to adjust the pH of the layer to 8-9. The mixture was stirred for 10 minutes. The ethyl acetate layer was separated again and was washed with a solution of common salt. (1Kg NaCl in 3L water). The mixture was stirred for 10 minutes. The ethyl acetate layer was separated once again and was concentrated under reduced pressure. The residue was taken up with n-hexane (10L) and was refluxed. The product was then cooled to 10 °C and was stirred for 30 minutes. The product obtained was filtered through a Nutsche filter and was washed with n-hexane (1L). The product, 1-(benzyloxy)-4-(chloromethyl)benzene, was dried at 40-42 °C over 5-6 hrs. Yield: 75-80% Purity: 90%.

### 3-(4-(benzyloxy)phenyl)-2-oxopropanal

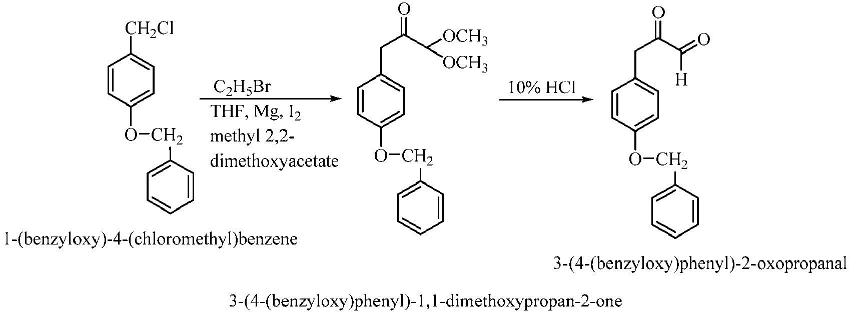

A 50L round bottom flask was charged with anhydrous tetrahydrofuran (7.5 L) and magnesium turnings (2.5 Kg) followed by iodine (2 grams) and ethyl bromide (10 mL). To this mixture, a solution of 1-(benzyloxy)-4-(chloromethyl)benzene (3.2 Kg) in anhydrous tetrahydrofuran (40 L) was added drop wise at 40-45 °C. The mixture was stirred for 1 h after which it was cooled to 20 °C. To this cooled mixture, a solution of methyl 2,2-dimethoxyacetate (2.5 Kg) in anhydrous tetrahydrofuran (2.5 L) was added at 20-38 °C. The mixture was stirred for 30 minutes at 40-42 °C. To the Grignard reaction mass, ammonium chloride solution (2.5 Kg ammonium chloride in 10L water) was added and this mixture was stirred for 20 minutes. The tetrahydrofuran layer was separated and was concentrated under reduced pressure. The product obtained was purified by column chromatography over silica gel. The purified product is heated to 60 °C along with 10L of 10% HCl for 3 hours to obtain 3-(4-(benzyloxy)phenyl)-2-oxopropanal. Yield : 65-70% Purity : 90%.

### 8-Benzyl-2-(4-hydroxybenzyl)-6-(4-hydroxyphenyl)imidazo[1,2-α]pyrazin-3(7H)-one (coelenterazine)

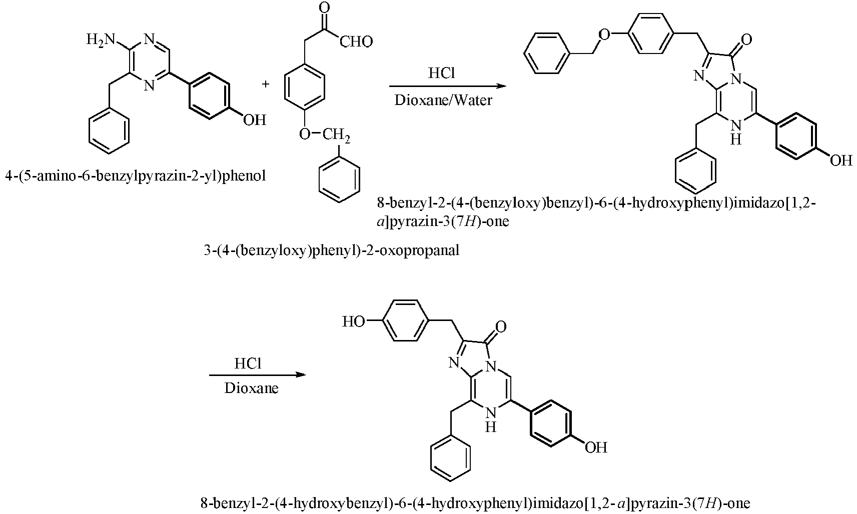

A 50 L round bottom flask was charged with 4-(5-amino-6-benzylpyrazin-2-yl)phenol (0.8 Kg) and 3-(4-(benzyloxy)phenyl)-2-oxopropanal (1.4 Kg) followed by 1,4-dioxane (10 L). The reaction mixture was stirred at room temperature for 30 minutes. Concentrated hydrochloric acid (1L) and water (1L) was added while nitrogen gas was being passed and the reaction mixture was heated to 80-85 °C for 24 hours in a nitrogen environment. After the completion of the reaction, the reaction mixture is cooled to 40 °C then activated carbon (200g) and activated silica gel (100g) are added and filtered.

Meanwhile, another 50 L round bottom flask was charged with HCl (15 L). Add slowly the above reaction mixture in to the HCl at 30-35 °C over 1 -1 ½ hrs. The mixture was stirred for 30 minutes at 30-35 °C. The product obtained was filtered through a Nutsche filter and was washed with toluene (1.5 L). The product was dried at 40-45 °C in 8-10 hrs.

The above product (1.5 Kg) was charged with dioxane-HCl (15 L) at room temperature. This mixture was stirred for 20 minutes and was then slowly heated to 60 °C. The reaction mixture was stirred at 60-62 °C for 12 hrs and it was then stirred for 3 hrs at 70-72 °C. The dioxane-HCl was removed completely under reduced pressure and the residue was cooled to 40 °C. The residue was then charged with ethyl acetate (5 L) and the mixture was stirred for 15 minutes. The ethyl acetate layer was decanted. The residue was charged with dichloromethane (5L) and this mixture was stirred for 35-40 minutes. Filter the compound through a Nutsche filter. To the filtrate dichloromethane (2.5 L) was added and this mixture was stirred for 25-30 minutes. The compound was filtered once again and the filtrate material was charged with n-hexane (4L). The mixture was stirred for 20-25 minutes. The compound, 8-benzyl-2-(4-hydroxybenzyl)-6-(4-hydroxyphenyl)imidazo[1,2-a]pyrazin-3(7H)-one, was filtered once again and was washed with n-hexane (1L). The product, 8-benzyl-2-(4-hydroxybenzyl)-6-(4-hydroxyphenyl)imidazo[1,2-a]pyrazin-3(7H)-one, was dried under reduced pressure at 40-45 °C over 7-8 hours. Yield : 80% Purity : 60-65%.

While illustrative embodiments have been illustrated and described, it will be appreciated that various changes can be made therein without departing from the scope of the disclosure.

## Claims

1. A method of making coelenterazine, comprising:
coupling 4-(5-amino-6-benzylpyrazin-2-yl)phenol (coelenteramine) with 3-(4-(benzyloxy)phenyl)-2-oxopropanal to provide 8-benzyl-2-(4-(benzyloxy)benzyl)-6-(4-hydroxyphenyl)imidazo[1,2-α]pyrazin-3(7H)-one; and
deprotecting the 8-benzyl-2-(4-(benzyloxy)benzyl)-6-(4-hydroxyphenyl)imidazo[1,2-α]pyrazin-3(7*H*)-one to provide 8-benzyl-2-(4-hydroxybenzyl)-6-(4-hydroxyphenyl)imidazo[1,2-α]pyrazin-3(7*H*)-one (coelenterazine).

2. The method of Claim 1, wherein coupling the 4-(5-amino-6-benzylpyrazin-2-yl)phenol (coelenteramine) with the 3-(4-(benzyloxy)phenyl)-2-oxopropanal is in a solvent mixture comprising dioxane, water, and HCl.

3. The method of Claim 2, wherein coupling the 4-(5-amino-6-benzylpyrazin-2-yl)phenol (coelenteramine) with the 3-(4-(benzyloxy)phenyl)-2-oxopropanal is at a temperature of 75 °C to 90 °C for 12 to 36 hours in an inert atmosphere.

4. The method of Claim 1, wherein deprotecting the 8-benzyl-2-(4-(benzyloxy)benzyl)-6-(4-hydroxyphenyl)imidazo[1,2-α]pyrazin-3(7*H*)-one comprises a first deprotection step of exposing the 8-benzyl-2-(4-(benzyloxy)benzyl)-6-(4-hydroxyphenyl)imidazo[1,2-α]pyrazin-3(7*H*)-one to HCl in an organic solvent comprising dioxane, and isolating and drying an intermediate deprotected product.

5. The method of claim 4, wherein the first deprotection step is at a temperature of 25 °C to 40 °C.

6. The method of Claim 4, further comprises a second deprotection step of exposing the dried intermediate deprotected product to HCl in an organic solvent comprising dioxane.

7. The method of claim 6, wherein second deprotection step comprises heating the intermediate deprotected product in HCl and the organic solvent to a temperature of about 70 °C to 75 °C for a duration of 12 to 24 hours to provide coelenterazine.

8. The method of Claim 1, wherein the coelenterazine is obtained in a yield of greater than 70% at a purity of from 55% to 70% relative to 4-(5-amino-6-benzylpyrazin-2-yl)phenol.

9. The method of Claim 1, further comprising making the 3-(4-(benzyloxy)phenyl)-2-oxopropanal by reacting 1-(benzyloxy)-4-(chloromethyl)benzene in two steps to provide 3-(4-(benzyloxy)phenyl)-2-oxopropanal.

10. The method of Claim 9, wherein making the 3-(4-(benzyloxy)phenyl)-2-oxopropanal does not include more than one palladium-catalyzed reaction.

11. The method of Claim 9, comprising a first step of reacting the 1-(benzyloxy)-4-(chloromethyl)benzene with methyl 2,2-dimethoxyacetate, ethyl bromide, magnesium, and a catalytic amount of iodine to provide 3-(4-(benzyloxy)phenyl)-1,1-dimethoxypropan-2-one.

12. The method of Claim 11, wherein the 3-(4-(benzyloxy)phenyl)-1,1-dimethoxypropan-2-one is purified by silica column chromatography.

13. The method of claim 12, further comprising a second step of reacting the 3-(4-(benzyloxy)phenyl)-1,1-dimethoxypropan-2-one with aqueous HCl to provide the 3-(4-(benzyloxy)phenyl)-2-oxopropanal.

14. The method of Claim 10, wherein the 3-(4-(benzyloxy)phenyl)-2-oxopropanal is isolated in a yield of 60 to 75% at a purity of 85 to 95% relative to 1-(benzyloxy)-4-(chloromethyl)benzene.

15. A method of making 3-(4-(benzyloxy)phenyl)-2-oxopropanal, comprising
providing 1-(benzyloxy)-4-(chloromethyl)benzene, and
reacting the 1-(benzyloxy)-4-(chloromethyl)benzene in two steps to provide 3-(4-(benzyloxy)phenyl)-2-oxopropanal.

16. The method of Claim 15, wherein the method does not include more than one palladium-catalyzed reaction.

17. The method of Claim 15, comprising a first step of reacting the 1-(benzyloxy)-4-(chloromethyl)benzene with methyl 2,2-dimethoxyacetate, ethyl bromide, magnesium, and a catalytic amount of iodine to provide 3-(4-(benzyloxy)phenyl)-1,1-dimethoxypropan-2-one.

18. The method of claim 17, wherein the 3-(4-(benzyloxy)phenyl)-1,1-dimethoxypropan-2-one is purified by silica column chromatography.

19. The method of claim 17, further comprising a second step of reacting the 3-(4-(benzyloxy)phenyl)-1,1-dimethoxypropan-2-one with aqueous HCl to provide the 3-(4-(benzyloxy)phenyl)-2-oxopropanal.

20. The method of Claim 15, wherein the 3-(4-(benzyloxy)phenyl)-2-oxopropanal is isolated in a yield of 60 to 75% at a purity of 85 to 95% relative to 1-(benzyloxy)-4-(chloromethyl)benzene.

## Patentansprüche

1. Verfahren zur Herstellung von Coelenterazin, umfassend:
Kuppeln von 4-(5-Amino-6-benzylpyrazin-2-yl)phenol (Coelenteramin) mit 3-(4-(Benzyloxy)phenyl)-2-oxopropanal zur Bereitstellung von 8-Benzyl-2-(4-(benzyloxy)benzyl)-6-(4-hydroxyphenyl)imidazo[1,2-α]pyrazin-3(7*H*)-on und
Entschützen von 8-Benzyl-2-(4-(benzyloxy)benzyl)-6-(4-hydroxyphenyl)imidazo[1,2-α]pyrazin-3(7*H*)-on zur Bereitstellung von 8-Benzyl-2-(4-hydroxybenzyl)-6-(4-hydroxyphenyl)imidazo[1,2-α]pyrazin-3(7*H*)-on (Coelenterazin).

2. Verfahren nach Anspruch 1, wobei das Kuppeln des 4-(5-Amino-6-benzylpyrazin-2-yl)phenols (Coelenteramin) mit dem 3-(4-(Benzyloxy)phenyl)-2-oxopropanal in einer Lösungsmittelmischung, die Dioxan, Wasser und HCl umfasst, erfolgt.

3. Verfahren nach Anspruch 2, wobei das Kuppeln des 4-(5-Amino-6-benzylpyrazin-2-yl)phenols (Coelenteramin) mit dem 3-(4-(Benzyloxy)phenyl)-2-oxopropanal bei einer Temperatur von 75 °C bis 90 °C über einen Zeitraum von 12 bis 36 Stunden in einer Inertatmosphäre erfolgt.

4. Verfahren nach Anspruch 1, wobei das Entschützen des 8-Benzyl-2-(4-(benzyloxy)benzyl)-6-(4-hydroxyphenyl)imidazo[1,2-α]pyrazin-3(7*H*)-ons einen ersten Entschützungsschritt des Einwirkenlassens von HCl auf das 8-Benzyl-2-(4-(benzyloxy)benzyl)-6-(4-hydroxyphenyl)imidazo[1,2-α]pyrazin-3(7*H*)-on in einem organischen Lösungsmittel, das Dioxan umfasst, und das Isolieren und Trocknen eines intermediären entschützten Produkts umfasst.

5. Verfahren nach Anspruch 4, wobei der erste Entschützungsschritt bei einer Temperatur von 25 °C bis 40 °C erfolgt.

6. Verfahren nach Anspruch 4, ferner umfassend einen zweiten Entschützungsschritt des Einwirkenlassens von HCl auf das getrocknete intermediäre entschützte Produkt in einem organischen Lösungsmittel, das Dioxan umfasst.

7. Verfahren nach Anspruch 6, wobei der zweite Entschützungsschritt das Erhitzen des intermediären geschützten Produkts in HCl und dem organischen Lösungsmittel auf eine Temperatur von etwa 70 °C bis 75 °C über einen Zeitraum von 12 bis 24 Stunden zur Bereitstellung von Coelenterazin umfasst.

8. Verfahren nach Anspruch 1, wobei das Coelenterazin in einer Ausbeute von mehr als 70 % bei einer Reinheit von 55 bis 70 %, bezogen auf 4-(5-Amino-6-benzylpyrazin-2-yl)phenol, erhalten wird.

9. Verfahren nach Anspruch 1, ferner umfassend das Herstellen des 3-(4-(Benzyloxy)phenyl)-2-oxopropanals durch Umsetzen von 1-(Benzyloxy)-4-(chlormethyl)benzol in zwei Schritten zur Bereitstellung von 3-(4-(Benzyloxy)phenyl)-2-oxopropanal.

10. Verfahren nach Anspruch 9, wobei das Herstellen des 3-(4-(Benzyloxy)phenyl)-2-oxopropanals nicht mehr als eine palladiumkatalysierte Umsetzung umfasst.

11. Verfahren nach Anspruch 9, umfassend einen ersten Schritt des Umsetzens des 1-(Benzyloxy)-4-(chlormethyl)benzols mit 2,2-Dimethoxyessigsäuremethylester, Ethylbromid, Magnesium und einer katalytischen Menge Iod zur Bereitstellung von 3-(4-(Benzyloxy)phenyl)-1,1 -dimethoxypropan-2-on.

12. Verfahren nach Anspruch 11, wobei das 3-(4-(Benzyloxy)phenyl)-1,1-dimethoxypropan-2-on durch Siliciumdioxid-Säulenchromatographie gereinigt wird.

13. Verfahren nach Anspruch 12, ferner umfassend einen zweiten Schritt des Umsetzens des 3-(4-(Benzyloxy)phenyl)-1,1-dimethoxypropan-2-ons mit wässrigem HCl zur Bereitstellung des 3-(4-(Benzyloxy)phenyl)-2-oxopropanals.

14. Verfahren nach Anspruch 10, wobei das 3-(4-(Benzyloxy)phenyl)-2-oxopropanal in einer Ausbeute von 60 bis 75 % bei einer Reinheit von 85 bis 95 %, bezogen auf 1-(Benzyloxy)-4-(chlormethyl)benzol, isoliert wird.

15. Verfahren zur Herstellung von 3-(4-(Benzyloxy)phenyl)-2-oxopropanal, umfassend das Bereitstellen von 1-(Benzyloxy)-4-(chlormethyl)benzol und das Umsetzen des 1-(Benzyloxy)-4-(chlormethyl)benzols in zwei Schritten zur Bereitstellung von 3-(4-(Benzyloxy)phenyl)-2-oxopropanal.

16. Verfahren nach Anspruch 15, wobei das Verfahren nicht mehr als eine palladiumkatalysierte Umsetzung umfasst.

17. Verfahren nach Anspruch 15, umfassend einen ersten Schritt des Umsetzens des 1-(Benzyloxy)-4-(chlormethyl)benzols mit 2,2-Dimethoxyessigsäuremethylester, Ethylbromid, Magnesium und einer katalytischen Menge Iod zur Bereitstellung von 3-(4-(Benzyloxy)phenyl)-1,1 -dimethoxypropan-2-on.

18. Verfahren nach Anspruch 17, wobei das 3-(4-(Benzyloxy)phenyl)-1,1-dimethoxypropan-2-on durch Siliciumdioxid-Säulenchromatographie gereinigt wird.

19. Verfahren nach Anspruch 17, ferner umfassend einen zweiten Schritt des Umsetzens des 3-(4-(Benzyloxy)phenyl)-1,1-dimethoxypropan-2-ons mit wässrigem HCl zur Bereitstellung des 3-(4-(Benzyloxy)phenyl)-2-oxopropanals.

20. Verfahren nach Anspruch 15, wobei das 3-(4-(Benzyloxy)phenyl)-2-oxopropanal in einer Ausbeute von 60 bis 75 % bei einer Reinheit von 85 bis 95 %, bezogen auf 1-(Benzyloxy)-4-(chlormethyl)benzol, isoliert wird.

## Revendications

1. Procédé de fabrication de coelentérazine, comprenant :
le couplage de 4-(5-amino-6-benzylpyrazin-2-yl)phénol (coelentéramine) avec le 3-(4-(benzyloxy)phényl)-2-oxopropanal pour produire la 8-benzyl-2-(4-(benzyloxy)benzyl)-6-(4-hydroxyphényl)imidazo[1,2-α]pyrazin-3(7*H*)-one ; et
la déprotection de la 8-benzyl-2-(4-(benzyloxy)benzyl)-6-(4-hydroxyphényl)imidazo[1,2-α]pyrazin-3(7*H*)-one pour produire la 8-benzyl-2-(4-hydroxybenzyl)-6-(4-hydroxyphényl)imidazo[1,2-α]pyrazin-3(7*H*)-one (coelentérazine).

2. Procédé selon la revendication 1, dans lequel le couplage du 4-(5-amino-6-benzylpyrazin-2-yl)phénol (coelentéramine) avec le 3-(4-(benzyloxy)phényl)-2-oxopropanal est effectué dans un mélange de solvants comprenant du dioxane, de l'eau et HCl.

3. Procédé selon la revendication 2, dans lequel le couplage du 4-(5-amino-6-benzylpyrazin-2-yl)phénol (coelentéramine) avec le 3-(4-(benzyloxy)phényl)-2-oxopropanal est à une température de 75 °C à 90 °C pendant 12 à 36 heures dans une atmosphère inerte.

4. Procédé selon la revendication 1, dans lequel la déprotection de la 8-benzyl-2-(4-(benzyloxy)benzyl)-6-(4-hydroxyphényl)imidazo[1,2-α]pyrazin-3(7*H*)-one comprend une première étape de déprotection consistant à exposer la 8-benzyl-2-(4-(benzyloxy)benzyl)-6-(4-hydroxyphényl)imidazo[1,2-α]pyrazin-3(7*H*)-one à HCl dans un solvant organique comprenant du dioxane, et isoler et sécher un produit déprotégé intermédiaire.

5. Procédé selon la revendication 4, dans lequel la première étape de déprotection est effectuée à une température de 25 °C à 40 °C.

6. Procédé selon la revendication 4, comprend en outre une deuxième étape de déprotection consistant à exposer le produit déprotégé intermédiaire séché à HCl dans un solvant organique comprenant du dioxane.

7. Procédé selon la revendication 6, dans lequel la deuxième étape de déprotection comprend le chauffage du produit déprotégé intermédiaire dans HCl et le solvant organique à une température d'environ 70 °C à 75 °C pendant une durée de 12 à 24 heures pour produire la coelentérazine.

8. Procédé selon la revendication 1, dans lequel la coelentérazine est obtenue avec un rendement supérieur à 70 % à une pureté de 55 % à 70 % par rapport au 4-(5-amino-6-benzylpyrazin-2-yl)phénol.

9. Procédé selon la revendication 1, comprenant en outre la fabrication du 3-(4-(benzyloxy)phényl)-2-oxopropanal par réaction du 1-(benzyloxy)-4-(chlorométhyl)benzène en deux étapes pour produire le 3-(4-(benzyloxy)phényl)-2-oxopropanal.

10. Procédé selon la revendication 9, dans lequel la fabrication du le 3-(4-(benzyloxy)phényl)-2-oxopropanal ne comprend pas plus d'une réaction catalysée par le palladium.

11. Procédé selon la revendication 9, comprenant une première étape de réaction du 1-(benzyloxy)-4-(chlorométhyl)benzène avec le 2,2-diméthoxyacétate de méthyle, le bromure d'éthyle, le magnésium, et une quantité catalytique d'iode pour produire la 3-(4-(benzyloxy)phényl)-1,1-diméthoxypropan-2-one.

12. Procédé selon la revendication 11, dans lequel la 3-(4-(benzyloxy)phényl)-1,1-diméthoxypropan-2-one est purifiée par chromatographie sur colonne de silice.

13. Procédé selon la revendication 12, comprenant en outre une deuxième étape de réaction de la 3-(4-(benzyloxy)phényl)-1,1-diméthoxypropan-2-one avec HCl aqueux pour produire le 3-(4-(benzyloxy)phényl)-2-oxopropanal.

14. Procédé selon la revendication 10, dans lequel le 3-(4-(benzyloxy)phényl)-2-oxopropanal est isolé avec un rendement de 60 à 75 % à une pureté de 85 à 95 % par rapport au 1-(benzyloxy)-4-(chlorométhyl)benzène.

15. Procédé de fabrication de 3-(4-(benzyloxy)phényl)-2-oxopropanal, comprenant la fourniture de 1-(benzyloxy)-4-(chlorométhyl)benzène, et
la réaction du 1-(benzyloxy)-4-(chlorométhyl)benzène en deux étapes pour produire le 3-(4-(benzyloxy)phényl)-2-oxopropanal.

16. Procédé selon la revendication 15, le procédé ne comprenant pas plus d'une réaction catalysée par le palladium.

17. Procédé selon la revendication 15, comprenant une première étape de réaction du 1-(benzyloxy)-4-(chlorométhyl)benzène avec le 2,2-diméthoxyacétate de méthyle, le bromure d'éthyle, le magnésium, et une quantité catalytique d'iode pour produire la 3-(4-(benzyloxy)phényl)-1,1-diméthoxypropan-2-one.

18. Procédé selon la revendication 17, dans lequel la 3-(4-(benzyloxy)phényl)-1,1-diméthoxypropan-2-one est purifiée par chromatographie sur colonne de silice.

19. Procédé selon la revendication 17, comprenant en outre une deuxième étape de réaction de la 3-(4-(benzyloxy)phényl)-1,1-diméthoxypropan-2-one avec HCl aqueux pour produire le 3-(4-(benzyloxy)phényl)-2-oxopropanal.

20. Procédé selon la revendication 15, dans lequel le 3-(4-(benzyloxy)phényl)-2-oxopropanal est isolé avec un rendement de 60 à 75 % à une pureté de 85 à 95 % par rapport au 1-(benzyloxy)-4-(chlorométhyl)benzène.
